Europäisches Patentamt
European Patent Office
Office européen des brevets

(19) (11) Publication number: **0 048 915**
**B1**

(12) **EUROPEAN PATENT SPECIFICATION**

(45) Date of publication of patent specification: **28.05.86**

(51) Int. Cl.$^4$: **C 07 D 501/59,** A 61 K 31/545

(21) Application number: **81107460.8**

(22) Date of filing: **19.09.81**

(54) **New cephem compounds, processes for preparation thereof and pharmaceutical composition comprising the same.**

(30) Priority: **26.09.80 US 190970**

(43) Date of publication of application: **07.04.82 Bulletin 82/14**

(45) Publication of the grant of the patent: **28.05.86 Bulletin 86/22**

(84) Designated Contracting States: **AT BE CH DE FR GB IT LI LU NL SE**

(58) References cited: **FR-A-2 383 951**

(73) Proprietor: **Fujisawa Pharmaceutical Co., Ltd.**
**3, Doshomachi 4-chome Higashi-ku**
**Osaka-shi, Osaka 541 (JP)**

(72) Inventor: **Takaya, Takao**
**No. 1-5-87, Suimeidai**
**Kawanishi (JP)**
Inventor: **Takasugi, Hisashi**
**No. 2-12-7, Kohamanishi**
**Suminoe-ku Osaka (JP)**
Inventor: **Chiba, Toshiyuki**
**No. 2-5-1-212, Nakamoto**
**Higashinari-ku Osaka (JP)**
Inventor: **Tsuji, Kiyoshi**
**No. 170, Hatacho**
**Kishiwada-shi (JP)**

(74) Representative: **Türk, Dietmar, Dr. rer. nat. et al**
**Redies, Redies, Türk & Gille Patentanwälte**
**Brucknerstrasse 20**
**D-4000 Düsseldorf 13 (DE)**

***The file contains technical information submitted after the application was filed and not included in this specification***

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 048 915 B1**

**Description**

The present invention relates to new cephem compounds and pharmaceutically acceptable salts thereof. More particularly, it relates to new cephem compounds and pharmaceutically acceptable salts thereof, which have antimicrobial activities, to processes for preparation thereof, to pharmaceutical composition comprising the same, usable in the treatment of infectious diseases in human being and animals.

A broad class of cephem compounds having a variety of substitution possibilities has been described in FR—A—2 383 951. However, there is no pointer to the specific cephem compounds in this reference.

It is one object of the present invention to provide new cephem compounds and pharmaceutically acceptable salts thereof, which are active against a number of pathogenic microorganisms and useful as antimicrobial agents, especially for oral administration.

Another object of the present invention is to provide processes for the preparation of new cephem compounds and pharmaceutically acceptable salts thereof.

A further object of the present invention is to provide pharmaceutical composition comprising, as active ingredients, said new cephem compounds and pharmaceutically acceptable salts thereof.

The object new cephem compounds are novel and can be represented by the following general formula:

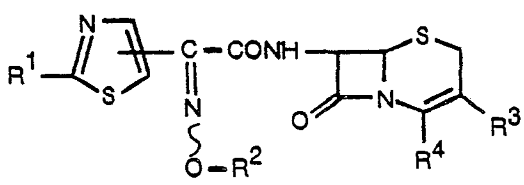

(I)

wherein

$R^1$ is amino or protected amino,

$R^2$ is carboxy($C_1$—$C_6$)alkylene or protected carboxy($C_1$—$C_6$)alkylene,

$R^3$ is halogen or $C_1$—$C_6$ alkoxy, and

$R^4$ is carboxy or protected carboxy.

According to the present invention the new cephem compounds (I) can be prepared by various processes which are illustrated in the following schemes.

PROCESS 1

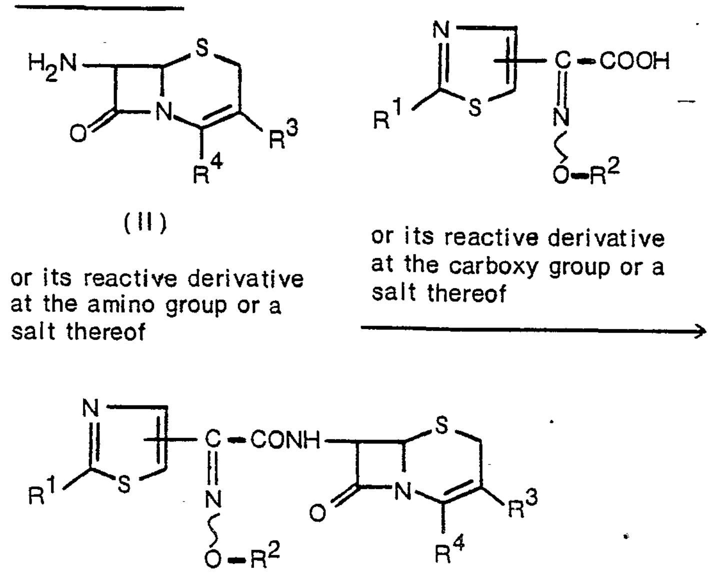


(II) (III) (I)

or a salt thereof

PROCESS 2

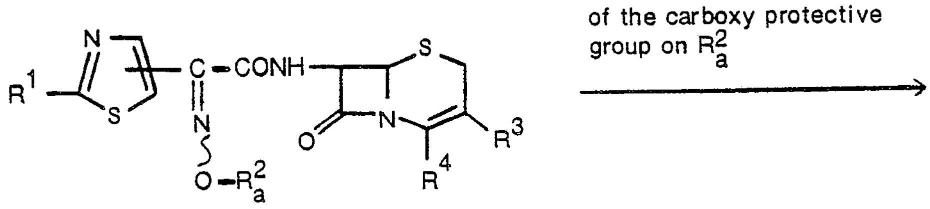


(Ia)

or a salt thereof

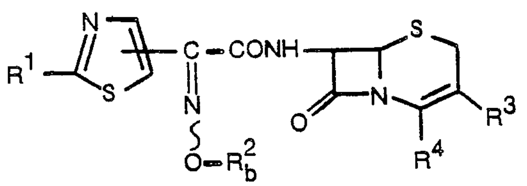

(Ib)

or a salt thereof

PROCESS 3

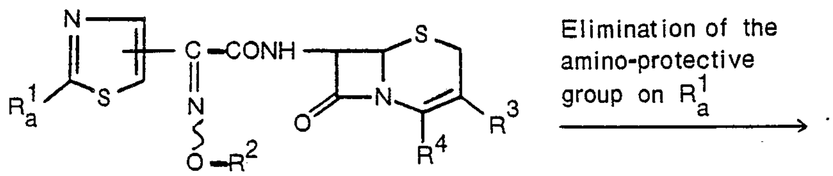


(Ic)

or a salt thereof

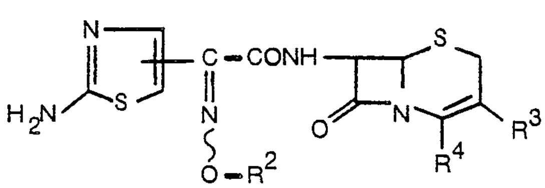

(Id)

or a salt thereof

PROCESS 4

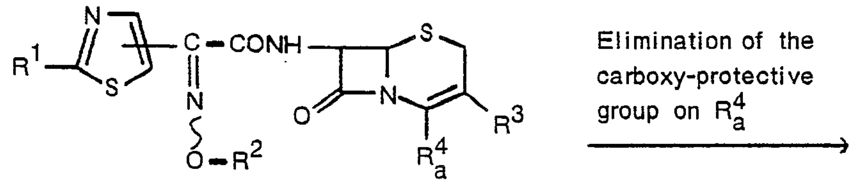


( Ie )

or a salt thereof

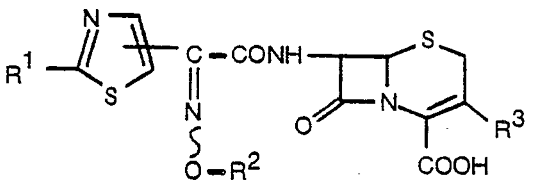

( If )

or a salt thereof

PROCESS 5

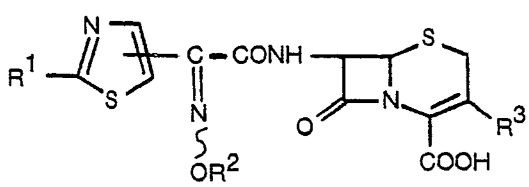

( If )

or a salt thereof

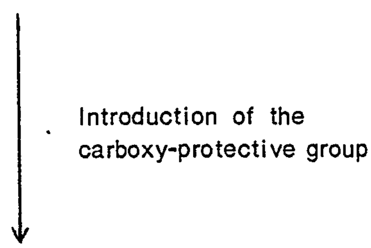


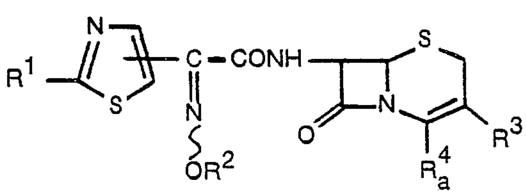

( Ie )

or a salt thereof

wherein

$R^1$, $R^2$, $R^3$ and $R^4$ are each as defined above,
$R^1_a$ is protected amino,
$R^2_a$ is protected carboxy($C_1$—$C_6$)alkyl,
$R^2_b$ is carboxy($C_1$—$C_6$)alkyl, and
$R^4_b$ is protected carboxy.

Regarding the object compounds (I), (Ia), (Ib), (Ic), (Id), (Ie), (If) and the starting compound (III), it is to be understood that said object and starting compounds include syn isomer, anti isomer and a mixture thereof. For example, with regard to the object compounds (I), syn isomer means one geometrical isomer having the partial structure represented by the following formula:

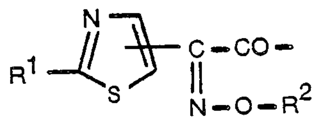

(wherein $R^1$ and $R^2$ are each as defined above) and anti isomer means the other geometrical isomer having the partial structure represented by the following formula:

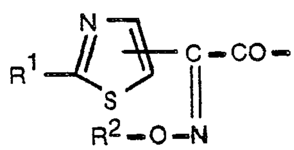

(wherein $R^1$ and $R^2$ are each as defined above).

Suitable pharmaceutically acceptable salts of the object compounds (I) are conventional non-toxic salts and include a metal salt such as an alkali metal salt (e.g. sodium salt, potassium salt) and an alkaline earth metal salt (e.g. calcium salt, magnesium salt), an ammonium salt, an organic base salt (e.g. trimethylamine salt, triethylamine salt, pyridine salt, picoline salt, dicyclohexylamine salt, N,N'-dibenzylethylenediamine salt), an organic acid salt (e.g. acetate, maleate, tartrate, methanesulfonate, benzenesulfonate, formate, toluenesulfonate), an inorganic acid salt (e.g. hydrochloride, hydrobromide, sulfate, phosphate), or a salt with an amino acid (e.g. arginine, aspartic acid, glutamic acid).

In the above and subsequent descriptions of the present specification, suitable examples and illustrations of the various definitions which the present invention include within the scope thereof are explained in details as follows.

Suitable "protected amino" may include an acylamino or an amino group substituted by a conventional protecting group such as ar($C_1$—$C_6$)alkyl which may have at least one suitable substituent(s), (e.g. benzyl, trityl).

Suitable acyl moiety in the terms "acylamino" may include aliphatic acyl group and acyl group containing an aromatic or heterocyclic ring. And, suitable examples of the said acyl may be $C_1$—$C_6$ alkanoyl (e.g. formyl, acetyl, propionyl, butyryl, isobutyryl, valeryl, isovaleryl, oxalyl, succinyl, pivaloyl); $C_1$—$C_6$ alkoxycarbonyl (e.g. methoxycarbonyl, ethoxycarbonyl, propoxycarbonyl, 1-cyclopropylethoxycarbonyl, isopropoxycarbonyl, butoxycarbonyl, tert-butoxycarbonyl, pentyloxycarbonyl, hexyloxycarbonyl); $C_1$—$C_6$ alkanesulfonyl (e.g. mesyl, ethanesulfonyl, propanesulfonyl, isopropanesulfonyl, butanesulfonyl); arenesulfonyl (e.g. benzenesulfonyl, tosyl, etc.); aroyl (e.g. benzoyl, toluoyl, xyloyl, naphthoyl, phthaloyl, indancarbonyl); ar($C_1$—$C_6$)alkanoyl (e.g. phenylacetyl, phenylpropionyl); and ar($C_1$—$C_6$)alkoxycarbonyl (e.g. benzyloxycarbonyl, phenethyloxycarbonyl). The acyl moiety as stated above may have at least one suitable substituent(s) such as halogen (chlorine, bromine, fluorine and iodine).

Preferable examples of acylamino may include lower alkanoylamino, and the most preferable one is formamido.

Suitable "protected carboxy" and "protected carboxy moiety" in the term "protected carboxy($C_1$—$C_6$)alkyl" may include an esterified carboxy, and suitable examples of the ester moiety in said esterified carboxy may be the ones such as $C_1$—$C_6$ alkyl ester (e.g. methyl ester, ethyl ester, propyl ester, isopropyl ester, butyl ester, isobutyl ester, pentyl ester, hexyl ester, 1-cyclopropylethyl ester) which may

have at least one suitable substituent(s), for example, $C_1$—$C_6$ alkanoyloxy($C_1$—$C_6$)alkyl ester (e.g. acetoxymethyl ester, propionyloxymethyl ester, butyryloxymethyl ester, valeryloxymethyl ester, pivaloyloxymethyl ester, 1 or 2-acetoxymethyl ester, 1 or 2-propionyloxyethyl ester, hexanoyloxymethyl ester), $C_1$—$C_6$ alkanesulfonyl($C_1$—$C_6$)alkyl ester (e.g. 2-mesylethyl ester) or mono(or di or tri)-halo($C_1$—$C_6$)alkyl ester (e.g. 2-iodoethyl ester, 2,2,2-trichloroethyl ester); up to $C_6$ alkenyl ester (e.g. vinyl ester, allyl ester); up to $C_6$ alkynyl ester (e.g. ethynyl ester, propynyl ester); ar($C_1$—$C_6$)alkyl ester which may have at least one suitable substituent(s) (e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-tert-butylbenzyl ester,); and aryl ester which may have at least one suitable substituent(s) (e.g. phenyl ester, 4-chlorophenyl ester, tolyl ester, tert-butylphenyl ester, xylyl ester, mesityl ester, cumenyl ester).

Preferable examples of the esterified carboxy as mentioned above may include $C_1$—$C_6$ alkoxycarbonyl (e.g. methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, tert-pentyloxycarbonyl, hexyloxycarbonyl, 1-cyclopropylethoxycarbonyl), mono- or di- or tri-phenyl($C_1$—$C_6$)alkoxycarbonyl (e.g benzyloxycarbonyl, benzhydryloxycarbonyl) which may have a nitro group (e.g. 4-nitrobenzyloxycarbonyl) and $C_1$—$C_6$ alkanoyloxy($C_1$—$C_6$)alkoxycarbonyl (e.g. acetoxymethoxycarbonyl, 1- or 2-propionyloxyethoxycarbonyl, 1- or 2-acetoxyethoxycarbonyl, 1- or 2- or 3-acetoxypropoxylcarbonyl, pivaloyloxymethoxycarbonyl, hexanoyloxymethoxycarbonyl), and the most preferable one is 4-nitrobenzyloxycarbonyl and pivaloyloxymethoxycarbonyl.

Suitable "$C_1$—$C_6$ alkyl moiety" in the terms "carboxy($C_1$—$C_6$)alkyl" and "protected carboxy($C_1$—$C_6$)alkyl" may include methyl, ethyl, propyl, isopropyl, butyl, t-butyl, pentyl and hexyl.

Preferable examples of carboxy($C_1$—$C_6$)alkyl may include carboxymethyl, 1-carboxyethyl, 2-carboxyethyl, 1-carboxypropyl, 2-carboxypropyl, 3-carboxypropyl, 4-carboxybutyl, 5-carboxypentyl, 6-carboxyhexyl, 1-carboxyisopropyl, 1-ethyl-1-carboxyethyl, 2-methyl-2-carboxypropyl, and the most preferable one is carboxymethyl.

Preferable examples of protected carboxy($C_1$—$C_6$)alkyl may include esterified carboxy($C_1$—$C_6$)alkyl, and more preferably $C_1$—$C_6$ alkoxycarbonyl($C_1$—$C_6$)alkyl (e.g. methoxycarbonylmethyl, ethoxycarbonylmethyl, propoxycarbonylmethyl, tert-butoxycarbonylmethyl, 2-ethoxycarbonylethyl, 2-ethoxycarbonylpropyl, 4-ethoxycarbonylbutyl, 1-tert-butoxycarbonylisopropyl, 1-tert-butoxycarbonyl-1-methylpropyl, 4-tert-butoxycarbonylbutyl, 5-tert-butoxycarbonylpentyl, 6-butoxycarbonylhexyl), mono- or di- or triphenyl($C_1$—$C_6$)alkoxycarbonyl($C_1$—$C_6$)alkyl (e.g. benzyloxycarbonylmethyl, benzhydryloxycarbonylmethyl, trityloxycarbonylmethyl, 1- or 2-benzyloxycarbonylethyl, 1- or 2- or 3-benzhydryloxycarbonylpropyl, 4-benzhydryloxycarbonylbutyl, 5-benzhydryloxycarbonylpentyl, 6-benzhydryloxycarbonylhexyl), and the most preferable one is tert-butoxycarbonylmethyl and benzhydryloxycarbonylmethyl.

Suitable "halogen" may include chlorine, bromine or iodine, and preferably chlorine or bromine, and the most preferably chlorine.

Suitable "$C_1$—$C_6$ alkoxy" may include methoxy, ethoxy, propoxy, isopropoxy, butoxy, pentyloxy and hexyloxy, and preferably methoxy.

The processes for preparing the object compounds (I) of the present invention are explained in details in the following.

Process 1

The object compounds (I) or a salt thereof can be prepared by reacting the compound (II) or its reactive derivative at the amino group or a salt thereof with the compound (III) or its reactive derivative at the carboxy group or a salt thereof.

Suitable reactive derivative at the amino group of the compound (II) may include Schiff's base type imino or its tautomeric enamine type isomer formed by the reaction of the compound (II) with a carbonyl compound such as aldehyde or ketone; a silyl derivative formed by the reaction of the compound (II) with a silyl compound such as bis(tri-methylsilyl)acetamide; a derivative formed by reaction of the compound (II) with phosphorus trichloride or phosgene.

Suitable salt of the compounds (II) and (III) may include an acid addition salt such as an organic acid salt (e.g. acetate, maleate, tartrate, benzene-sulfonate, toluenesulfonate) or an inorganic acid salt (e.g. hydrochloride, hydrobromide, sulfate, phosphate); a metal salt such as alkali metal or alkaline earth metal salt (e.g. sodium salt, potassium salt, calcium salt, magnesium salt); ammonium salt; and an organic amine salt (e.g. triethylamine salt, dicyclohexylamine salt).

Suitable reactive derivative at the carboxy group of the compound (III) may include an acid halide, an acid azide and acid anhydride, an activated amide and an activated ester. The suitable example may be an acid chloride, and acid bromide; a mixed acid anhydride with an acid such as substituted phosphoric acid (e.g. dialkylphosphoric acid, phenylphosphoric acid, diphenylphosphoric acid, dibenzylphosphoric acid, halogenated phosphoric acid, dialkyl phosphorous acid, sulfurous acid, thiosulfuric acid, sulfuric acid, alkyl carbonic acid (e.g methyl carbonate, ethyl carbonate), aliphatic carboxylic acid (e.g. pivalic acid, pentanoic acid, isopentanoic acid, 2-ethylbutyric acid or trichloroacetic acid) or aromatic carboxylic acid (e.g benzoic acid); a symmetrical acid anhydride; an activated amide with imidazole, 4-substituted imidazole, dimethylpyrazole, triazole or tetrazole; or an activated ester (e.g.

cyanomethyl ester, methoxymethyl ester, dimethyliminomethyl $[(CH_3)_2\overset{+}{N}=CH—]$ ester, vinyl ester, propargyl ester, p-nitrophenyl ester, 2,4-dinitrophenyl ester, trichlorophenyl ester, pentachlorophenyl ester, mesyl-phenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester), or an ester with a N-hydroxy compound (e.g. N,N-dimethylhydroxylamine, 1-hydroxy-2-(1H)-pyridone, N-hydroxysuccinimide, N-hydroxyphthalimide, 1-hydroxy-6-chloro-1H-benzotriazole). These reactive derivatives can optionally be selected from them according to the kind of the compound (III) to be used.

The reaction is usually carried out in a conventional solvent such as water, acetone, dioxane, acetonitrile, chloroform, methylene chloride, ethylene chloride, tetrahydrofuran, ethyl acetate, N,N-dimethylformamide, pyridine or any other organic solvents which do not adversely influence the reaction. These conventional solvents may also be used in a mixture with water.

When the compound (III) is used in free acid form or its salt form in the reaction, the reaction is preferably carried out in the presence of a conventional condensing agent such as N,N'-dicyclohexylcarbodiimide; N-cycylhexyl-N'-morpholinoethylcarbodiimide; N-cyclohexyl-N'-(4-diethylaminocyclohexyl)carbodiimide; N,N'-diethylcarbodiimide, N,N'-diisopropylcarbodiimide; N-ethyl-N'-(3-dimethylaminopropyl)carbodiimide; N,N-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; thionyl chloride; oxalyl chloride; triphenylphosphine; 2-ethyl-7-hydroxybenzisoxazolium salt; 2-ethyl-5-(m-sulfophenyl)isoxazolium hydroxide intra-molecular salt; 1-(p-chlorobenzenesulfonyloxy)-6-chloro-1H-benzotriazole; or so-called Vilsmeier reagent prepared by the reaction of dimethylformamide with e.g. thionyl chloride, phosgene and phosphorus oxychloride.

The reaction may also be carried out in the presence of an inorganic or organic base such as an alkali metal bicarbonate (e.g. sodium bicarbonate, potassium bicarbonate), an alkali metal carbonate (e.g. sodium carbonate, potassium carbonate), an alkaline earth metal carbonate (e.g. calcium carbonate), tri($C_1$—$C_6$)alkylamine (e.g. trimethylamine, triethylamine), pyridine, N-($C_1$—$C_6$)alkylmorphorine or N,N-di($C_1$—$C_6$)alkylbenzylamine. The reaction temperature is not critical, and the reaction is usually carried out under cooling to under warming.

In the present reaction, a syn isomer of the object compounds (I) can be obtained preferably by conducting the present reaction of the compound (II) with the corresponding syn isomer of the starting compound (III), for example, in the presence of a Vilsmeier reagent as mentioned above and under around neutral condition.

Process 2:

The object compound (Ib) or a salt thereof can be prepared by subjecting the compound (Ia) or a salt thereof to elimination reaction of the carboxy protective group on $R_a^2$.

Suitable salt of the compound (Ia) can be referred to the one exemplified for the compounds (I).

The present elimination reaction can be carried out in a similar manner to that of aftermentioned Process 4.

The present invention includes, within its scope, the cases that another protected carboxy and/or protected amino group(s) are converted into the corresponding free carboxy and/or amino group(s) during the reaction or the post-treating step of the present process.

Process 3:

The object compound (Id) or a salt thereof can be prepared by subjecting the compound (Ic) or a salt thereof to elimination reaction of the amino-protective group on $R_a^1$.

Suitable salt of the compound (Ic) can be referred to the metal salt, ammonium salt and organic amine salt exemplified for the compound (II).

The elimination reaction is carried out in accordance with a conventional method such as hydrolysis; reduction; or a method treating the compound (Ic) wherein $R_a^1$ is acylamino with iminohalogenating agent, iminoetherifying agent and then, if necessary, hydrolyzing the resultant compound. The hydrolysis may include a method using an acid or base or hydrazine. These methods may be selected depending on the kind of the protective groups to be eliminated.

Among these methods, hydrolysis using an acid is one of the most common and preferable method for eliminating the protective groups such as substituted or unsubstituted alkoxycarbonyl, for example, $C_1$—$C_6$ alkoxycarbonyl (e.g. tert-butoxycarbonyl, tert-pentyloxycarbonyl), $C_1$—$C_6$ alkanoyl (e.g. formyl, acetyl), cycloalkoxycarbonyl, substituted or unsubstituted aralkoxycarbonyl, for example, mono- or di or triphenyl($C_1$—$C_6$)alkoxycarbonyl (e.g. benzyloxycarbonyl, benzhydryloxycarbonyl), aralkyl (e.g. benzyl, trityl), substituted phenylthio, substituted aralkylidene, substituted alkylidene or substituted cycloalkylidene.

Suitable acid includes an organic or inorganic acid such as formic acid, trifluoroacetic acid, benzenesulfonic acid, p-tolouenesulfonic acid and hydrochloric acid, and the most suitable acid is an acid which can easily be removed from the reaction mixture by a conventional manner such as distillation under reduced

pressure, for example, formic acid, trifluoroacetic acid and hydrochloric acid. The acids can be selected according to the kind of the protective group to be eliminated. When the elimination reaction is conducted with an acid, it can be carried out in the presence or absence of a solvent. Suitable solvent includes water, a conventional organic solvent which does not adversely influence the reaction such as methanol, ethanol, propanol, tetrahydrofuran, or a mixture thereof.

The elimination reaction using trifluoroacetic acid may be carried out in the presence of anisole. The hydrolysis using hydrazine is commonly applied for eliminating a phthaloyl, succinyl type amino-protective group.

The elimination using base is used for eliminating an acyl group such as trifluoroacetyl. Suitable base may include an inorganic base and an organic base as aforementioned.

The reductive elimination is generally applied for eliminating the protective group, for example, haloalkoxycarbonyl (e.g. trichloroethoxycarbonyl), substituted or unsubstituted aralkoxycarbonyl (e.g. benzyloxycarbonyl) and 2-pyridylmethoxycarbonyl. Suitable method for this reduction may include, for example, reduction with an alkali metal borohydride (e.g. sodium borohydride, sodium cyanoborohydride), reduction with a combination of a metal (e.g. tin, zinc, iron) or the said metal together with a metal salt compound (e.g. chromous chloride, chromous acetate,) and an organic or inorganic acid (e.g. formic acid, acetic acid, propionic acid, hydrochloric acid); and catalytic reduction. Suitable catalyst in catalytic reduction includes a conventional one, for example, Raney nickel, platinum oxide and palladium carbon.

Among the protective groups, the acyl group can generally be eliminated by hydrolysis. Especially, halogen substituted-alkoxycarbonyl and 8-quinolyloxycarbonyl groups are usually eliminated by treating with a heavy metal such as copper or zinc.

Among the protective groups, the acyl group can also be eliminated by treating with an iminohalogenating agent (e.g. phosphorus oxychloride) and an iminoetherifying agent such as $C_1$—$C_6$ alkanol (e.g. methanol, ethanol), if necessary, followed by hydrolysis.

The reaction temperature is not critical and may suitably be selected in accordance with the kind of the amino protective group and the elimination method as mentioned above, and the reaction is preferably carried out under a mild condition such as under cooling or at slightly elevated temperature.

The present invention includes, within its scope, the cases that another protected amino and/or protected carboxy group(s) are converted into the corresponding free amino and/or the free carboxy group(s) during the reaction or the post-treating step of the present process.

Process 4:

The object compound (If) or a salt thereof can be prepared by subjecting the compound (Ie) or a salt thereof to elimination reaction of the carboxy-protective group on $R^4_a$.

Suitable salt of the compound (Ie) can be referred to the acid addition salt exemplified for the compound (II).

In the present elimination reaction, all conventional methods used in an elimination reaction of the carboxy-protective group, for example, hydrolysis, reduction and elimination using Lewis acid, are applicable. When the carboxy-protective group is an ester, it can be eliminated by hydrolysis or elimination using Lewis acid. The hydrolysis is preferably carried out in the presence of a base or an acid. Suitable base may include an inorganic base and an organic base as aforementioned.

Suitable acid may include an organic acid (e.g. formic acid, acetic acid, propionic acid) and an inorganic acid (e.g. hydrochloric acid, hydrobromic acid, sulfuric acid).

The present hydrolysis is usually carried out in an organic solvent such as those given in Hydrolysis for Process 3, water or a mixed solvent thereof.

The reaction temperature is not critical, and it may suitably be selected in accordance with the kind of the carboxy-protective group and the elimination method.

The elimination using Lewis acid is preferable to eliminate substituted or unsubstituted ar($C_1$—$C_6$)alkyl ester and carried out by reacting the compound (e) or a salt thereof with Lewis acid such as boron trihalide (e.g. boron trichloride, boron trifluoride,), titanium tetrahalide (e.g. titanium tetrachloride, titanium tetrabromide), tin tetrahalide (e.g. tin tetrachloride, tin tetrabromide), aluminum halide (e.g. aluminum chloride, aluminum bromide) or trihaloacetic acid (e.g. trichloroacetic acid, trifluoroacetic acid). This elimination reaction is preferably carried out in the presence of cation trapping agents (e.g. anisole, phenol) and is usually carried out in a solvent such as nitroalkane (e.g. nitromethane, nitroethane), alkylene halide (e.g. methylene chloride, ethylene chloride), diethyl ether, carbon disulfide or any other solvent which does not adversely affect the reaction. These solvents may be used as a mixture thereof. The reaction temperature is not critical, and the reaction is usually carried out under cooling, at ambient temperature or under warming.

The reductive elimination can be applied preferably for elimination of the protective group such as halo($C_1$—$C_6$)alkyl (e.g. 2-iodoethyl, 2,2,2-trichloroethyl) ester or ar($C_1$—$C_6$)alkyl which may have nitro (e.g. benzyl, p-nitrobenzyl) ester.

The reduction method applicable for the elimination reaction may include, for example, reduction by using a combination of a metal (e.g. zinc, zinc amalgam,) or a salt of chromium compound (e.g. chromous chloride, chromous acetate) and an organic or inorganic acid (e.g. acetic acid, propionic acid, hydrochloric

acid); and conventional catalytic reduction in the presence of a conventional metallic catalyst (e.g. palladium carbon, Raney nickel).

The reductive elimination is usually carried out in the presence of a solvent which does not adversely influence the reaction such as methanol, ethanol, propanol, diethyl ether, or a mixture thereof. The reaction temperature is not critical and the reaction is usually carried out under cooling to warming.

The present elimination reaction of the carboxy-protective group includes, within its scope, the cases that protected amino group in the compound (Ie) is transformed into free amino group according to reaction conditions and kinds of the protective groups in the course of the reaction and/or in post-treatment of the reaction.

Process 5:

The object compound (Ie) or a salt thereof can be prepared by subjecting the compound (If) or a salt thereof to introduction reaction of the carboxy-protective group.

The introducing agent of a carboxy-protective group to be used in this reaction may include a conventional esterifying agent which can introduce "ester moiety" as aforementioned into the carboxy group of the compound (If) such as the corresponding alcohol or its reactive equivalent (e.g. halide such as chloro, bromide and iodide, sulfonate, sulfate, diazo compound).

The reaction can also be carried out in the presence of a base, and suitable examples thereof are the same as those given in the explanation of Process 1, and can preferably be carried out in the presence of metal iodide (e.g. sodium iodide).

This reaction is usually carried out in a conventional solvent which does not adversely influence the reaction such as N,N-dimethylformamide, tetrahydrofuran, dioxane, methanol, ethanol, or a mixture thereof.

The reaction temperature is not critical, and the reaction is usually carried out under cooling to a somewhat elevated temperature.

In case that the alcohol is used as the introducing agent of a carboxy-protective group, the reaction can be carried out in the presence of a condensing agent as illustrated in Process 1.

The present invention includes, within its scope, the cases that the one type of tautomeric isomers is converted into the other type of isomer during the reaction and/or the post-treating step of the each process.

In case that the object compounds (I) are obtained in a form of the free acid at the 4-position and/or the oxime portion, and/or in case that the compounds (I) have free amino group, it may be transformed into its pharmaceutically acceptable salt as aforementioned by a conventional method.

The object compounds (I) and pharmaceutically acceptable salt thereof of the present invention are novel compounds which exhibit high antimicrobial activity and inhibit the growth of a wide variety of pathogenic microorganisms including Gram-positive and Gram-negative microorganisms, and are useful as anti-microbial agents, especially for oral administration. For therapeutic purpose, the compounds according to the present invention can be used in a form of pharmaceutical preparation which contain said compounds, as active ingredient, in admixture with pharmaceutically acceptable carriers such as an organic or inorganic solid or liquid excipient suitable for oral, parenteral or external administration. The pharmaceutical preparations may be capsules, tablets, dragees, ointments or suppositories, solutions, suspensions and emulsions. If desired, there may be included in the above preparations auxiliary substances, stabilizing agents wetting or emulsifying agents, buffers and other commonly used additives.

While the dosage of the compounds (I) will vary from and depend upon the age and condition of the patient, an average single dose of about 10 mg, 50 mg, 100 mg, 250 mg, 500 mg, and 1000 mg of the compounds according to the present invention was proved to be effective for treating infectious diseases caused by pathogenic microorganisms. In general, amounts between 1 mg/body and about 6,000 mg/body or even more may be administered per day.

In order to illustrate the usefulness of the object compounds (I), antimicrobial activities or urinary or biliary excretion of some representative compounds of the present invention are shown below.

(1) Antimicrobial Activities:

*Test Method*

In vitro antibacterial activity was determined by the two-fold agar-plate dilution method as described below.

One loopful of an overnight culture of each test strain in Trypticase-soy broth ($10^8$ viable cells per ml) was streaked on heart infusion agar (HI-agar) containing graded concentrations of representative test compound, and the minimal inhibitory concentration (MIC) was expressed in terms of μg/ml after incubation at 37°C for 20 hours.

*Test compound*

7-[2-carboxymethylimino-2-(2-aminothiazol-4-yl)-acetamido]-3-chloro-3-cephem-4-carboxylic acid (syn isomer).

*Test results*

M.I.C. (μg/ml)

| Test Microorganisms \ Compound | (1) |
|---|---|
| Proteus vulgaris IAM-1025 | 0.1 |
| Escherichia coli NIHJ JC-2 | 0.39 |
| Klebsiella pneumoniae 7 | 0.05 |
| Proteus mirabilis 1 | 0.025 |
| Escherichia cloacae 60 | 25.0 |

(2) Urinary and Biliary excretions:

*Test Method*

① Urinary excretion:—

The test compound (100 mg/kg) was given orally to groups of 3 rats, and urinary samples were collected at 0 to 24 hours.

② Biliary excretion:—

Groups of 3 rats anesthetized with intraperitoneal pentobarbital were fixed in the supine position, and a polyethylene cannula was inserted into the bile duct. Bile samples were collected at 0 to 24 hours after oral administration of 100 mg/kg of the test compound. The antibiotic levels in the bile samples were assayed with the standard solutions prepared with M/15 phosphate buffer at pH 7.0 and biliary recovery was calculated.

*Test Compounds*

(A) 7-[2-Carboxymethoxyimino-2-(2-aminothiazol-4-yl)-acetamido]-3-methoxy-3-cephem-4-carboxylic acid (syn isomer) (hereinafter referred to as Compound A).

(B) 7-[2-Carboxymethoxyimino-2-(2-aminothiazol-4-yl)-acetamido]-3-chloro-3-cephem-4-carboxylic acid (syn isomer) (hereinafter referred to as Compound B).

*Test Results*

| Compounds | Urinary excretion (%) | biliary excretion (%) | Total (%) |
|---|---|---|---|
| A | 41.62 | 17.98 | 59.6 |
| B | 35.80 | 15.52 | 51.32 |

The following Examples are given for the purpose of illustrating the present invention in more detail.

Example 1

Dry tetrahydrofuran (20 ml) and 2-(tert-butoxycarbonylmethoxyimino)-2-(2-formamidothiazol-4-yl)acetic acid (syn isomer, 2.1 g) were added to a Vilsmeier reagent, which was prepared from dry N,N-dimethylformamide (0.6 g), dry ethyl acetate (2.4 ml) and phosphorus oxychloride (1.4 g) in a usual manner, and the resulting mixture was stirred at −3 to 3°C for 30 minutes to give a solution containing the activated acid.

Dry ethyl acetate (50 ml) and N-(trimethylsilyl)acetamide (5.4 g) were added to 4-nitrobenzyl 7-amino-3-chloro-3-cephem-4-carboxylate hydrochloride (2.5 g), and stirred at 40°C for 20 minutes. To the solution was added the solution containing the activated acid at −10°C and stirred at −10 to −5°C for 30 minutes. Water (40 ml) was added to the resultant solution and allowed to stand at room temperature. The organic layer was separated, washed with a saturated solution of sodium bicarbonate twice and saturated solution of sodium chloride subsequently, and dried over magnesium sulfate. The solution was concentrated to dryness and triturated with diisopropyl ether. The precipitates were collected by filtration and dried to give 4 - nitrobenzyl 7 - [2 - (tert - butoxycarbonylmethoxyimino) - 2 - (2 - formamidothiazol - 4 -

yl)acetamido] - 3 - chloro - 3 - cephem - 4 - carboxylate (syn isomer, 3.9 g).

IR (Nujol): 1780, 1730, 1680, 1640 $cm^{-1}$

NMR (DMSO-$d_6$, δ): 1.45 (9H, s), 3.93 (2H, q, J=16.0Hz), 4.63 (2H, s), 5.26 (1H, d, J=5.0Hz), 5.48 (2H, s), 5.96 (1H, dd, J=5.0Hz, 8.0Hz), 7.45 (1H, s), 7.72 (2H, d, J=9.0Hz), 8.28 (2H, d, J=9.0Hz), 8.55 (1H, s), 9.72 (1H, d, J=8.0Hz).

Example 2

4-Nitrobenzyl 7-amino-3-methoxy-3-cephem-4-carboxylate hydrochloride (4.02 g) and 2-(tert-butoxycarbonylmethoxyimino)-2-(2-formamidothiazol-4-yl)acetic acid (syn isomer), 3.62 g) were treated in a similar manner to that of Example 1 to give 4 - nitrobenzyl 7 - [2 - (tert-butoxycarbonylmethoxyimino) - 2 - (2 - formamidothiazol - 4 - yl)acetamio] - 3 - methoxy - 3 - cephem - 4 - carboxylate (syn isomer, 6.8 g).

IR (Nujol): 3400—3150, 1770, 1720, 1680, 1600, 1530 $cm^{-1}$

NMR (DMSO-$d_6$, δ): 1.46 (9H, s), 3.70 (2H, broad s), 3.86 (3H, s), 4.66 (2H, s), 5.26 (1H, d, J=5Hz), 5.36 (2H, m), 5.70 (1H, d, d, J=5.8Hz), 7.53 (1H, s), 7.65 (2H, d, J=8Hz), 8.20 (2H, d, J=8Hz), 8.50 (1H, s), 9.56 (1H, d, J=8Hz).

Example 3

Methanol (40 ml), tetrahydrofuran (20 ml) and acetic acid (0.3 ml) were added to 4 - nitrobenzyl 7 - [2 - (tert-butoxycarbonylmethoxyimino) - 2 - (2 - formamidothiazol - 4 - yl)acetamido] - 3 - chloro - 3 - cephem - 4 - carboxylate (syn isomer, 3.8 g). 10% Palladium carbon (1.9 g) containing water (3 ml) was added to the solution, and subjected to catalytic reduction for 4 hours. After removal of palladium carbon by filtration, the filtrate was concentrated in vacuo. To the residue were added ethyl acetate and water, and the solution was adjusted to pH 7.5 with a saturated solution of sodium bicarbonate. After the aqueous layer was separated, ethyl acetate was added to the aqueous layer, and adjusted to pH 2.0 with 10% hydrochloric acid. The ethyl acetate layer was separated. The organic solution was washed with a saturated solution of sodium chloride, dried over magnesium sulfate and concentrated in vacuo. The residue was triturated with diisopropyl ether, and then the precipitates were collected by filtration and dried to give 7 - [2 - (tert-butoxycarbonylmethoxyimino) - 2 - (2 - formamidothiazol - 4 - yl) - acetamido] - 3 - chloro - 3 - cephem - 4 - carboxylic acid (syn isomer, 2.31 g).

IR (Nujol): 3160, 1780, 1720, 1670 $cm^{-1}$

Example 4

4 - Nitrobenzyl 7 - [2 - (tert-butoxycarbonylmethoxyimino) - 2 - (2 - formamidothiazol - 4 - yl)acetamido] - 3 - methoxy - 3 - cephem - 4 - carboxylate (syn isomer, 6.8 g), methanol (60 ml) tetrahydrofuran (60 ml), acetic acid (6 ml), water (10 ml) and 10% palladium carbon (3.5 g) were treated in a similar manner to that of Example 3 to give 7 - [2 - (tert-butoxycarbonylmethoxyimino) - 2 - (2 - formamidothiazol - 4 - yl)acetamido] - 3 - methoxy - 3 - cephem - 4 - carboxylic acid (syn isomer, 3.8 g).

IR (Nujol): 3250, 1770, 1680, 1590, 1540 $cm^{-1}$

NMR (DMSO-$d_6$; δ): 1.47 (9H, s), 3.63 (2H, s), 3.78 (3H, s), 4.65 (2H, s), 5.18 (1H, d, J=5Hz), 5.62 (1H, d, d, J=5.8Hz), 7.53 (1H, s), 8.53 (1H, s), 9.56 (1H, d, J=8Hz).

Example 5

Methonol (15 ml) and conc. hydrochloric acid (0.8 g) were added to 7 - [2 - (tert-butoxycarbonylmethoxyimino) - 2 - (2 - formamidothiazol - 4 - yl)acetamido] - 3 - chloro - 3 - cephem - 4 - carboxylic acid (syn isomer, 2.2 g), and stirred at room temperature for 2.5 hours. To the resultant solution were added ethyl acetate (50 ml) and water (50 ml), and then the solution was adjusted to pH 7.5 with a saturated solution of sodium bicarbonate. After separating the aqueous layer, the aqueous solution was saturated with sodium chloride, and adjusted to pH 3.0 with 10% hydrochloric acid. After adding water, the solution was extracted with ethyl acetate. The extract was washed with a saturated solution of sodium chloride, dried over magnesium sulfate and concentrated in vacuo. The residue was triturated with diisopropyl ether and the precipitates were collected by filtration and dried to give 7 - [2 - (tert-butoxycarbonylmethoxyimino) - 2 - (2 - aminothiazol - 4 - yl)acetamido] - 3 - chloro - 3 - cephem - 4 - carboxylic acid (syn isomer, 1.72 g).

IR (Nujol): 1770, 1720, 1660, 1610 $cm^{-1}$

NMR (DMSO-$d_6$, δ): 1.44 (9H, s), 3.84 (2H, q, J=18.0Hz), 4.58 (2H, s), 5.29 (1H, d, J=5.0Hz), 5.85 (1H, dd, J=5.0Hz, 8.0Hz), 6.80 (1H, s), 7.27 (2H, broad-s), 9.57 (1H, d, J=8.0Hz).

Example 6

7 - [2 - (tert-butoxycarbonylmethoxyimino) - 2 - (2 - formamidothiazol - 4 - yl)acetamido] - 3 - methoxy - 3 - cephem - 4 - carboxylic acid (syn isomer, 3.7 g), methanol (40 ml) and conc. hydrochloric acid (2.1 ml) were treated in a similar manner to that of Example 5 to give 7 - [2 - (tert - butoxycarbonylmethoxyimino) - 2 - (2 - aminothiazol - 4 - yl)acetamido] - 3 - methoxy - 3 - cephem - 4 - carboxylic acid (syn isomer, 2.8 g).

IR (Nujol): 3350—3150, 1760, 1720 (shoulder), 1670, 1620, 1530 $cm^{-1}$

NMR (DMSO-$d_6$, δ): 1.45 (9H, s), 3.6 (2H, s), 3.78 (3H, s), 4.58 (2H, s), 5.15 (1H, d, J=5Hz), 5.57 (1H, d,d, J=5, 8Hz), 6.9 (1H, s), 9.43 (1H, d, J=8Hz)

Example 7

Methylene chloride (3.0 ml), anisole (1.6 ml) and trifluoroacetic acid (6.4 ml) were added to 7 - [ - 2 - (tert - butoxycarbonylmethoxyimino) - 2 - (2 - aminothiazol - 4 - yl)acetamido] - 3 - chloro - 3 - cephem - 4 - carboxylic acid (syn isomer, 1.6 g), and stirred at room temparature for 1.5 hours. To the resultant solution was added diisopropyl ether and triturated. The precipitates were collected by filtration and washed with diisopropyl ether. After water and ethyl acetate were added to the residue, the solution was adjusted to pH 7.5 with a saturated solution of sodium bicarbonate and the aqueous solution was separated. The aqueous solution was washed with ethyl acetate, and adjusted to pH 2.2 with diluted hydrochloric acid. The precipitates were collected by filtration, washed with water and dried over phosphorus pentoxide to give 7 - [2 - carboxymethoxyimino - 2 - (2 - aminothiazol - 4 - yl)acetamido] - 3 - chloro - 3 - cephem - 4 - carboxylic acid (syn isomer, 0.71 g).

IR (Nujol): 3250, 1770, 1660 $cm^{-1}$

NMR (DMSO-$d_6$, δ): 3.86 (2H, q, J=18.0Hz, 4.63 (2H, s), 5.29 (1H, d, J=4.5Hz), 5.86 (1H, dd, J=4.5Hz, 8.0Hz), 6.83 (1H, s), 9.59 (1H, d, J=8.0Hz)

Example 8

7 - [2 - (tert - butoxycarbonylmethoxyimino) - 2 - (2 - aminothiazol - 4 - yl)acetamido] - 3 - methoxy - 3 - cephem - 4 - carboxylic acid (syn isomer, 2.7 g), methylene chloride (10 ml), anisole (2.2 g) and trifluoroacetic acid (12 g) were treated in a similar manner to that of Example 7 to give 7 - [2 - carboxymethoxyimino - 2 - (2 - aminothiazol - 4 - yl)acetamido] - 3 - methoxy - 3 - cepham - 4 - carboxylic acid (syn isomer, 1.1 g).

IR (Nujol): 1760, 1660 $cm^{-1}$

NMR (DMSO-$d_6$, δ): 3.67 (2H, s), 3.83 (3H, s), 4.73 (2H, s), 5.20 (1H, d, J=5Hz), 5.62 (1H, d,d,J=5.8Hz), 7.05 (1H, s), 9.60 (1H, d, J=8Hz)

Example 9

Vilsmeier reagent was prepared from N,N-dimethylformamide (1.5 g) and phosphorus oxychloride (3.2 g) in ethyl acetate (6 ml) in a usual manner. To this reagent in ethyl acetate (80 ml) was added 2 - (benzhydryloxycarbonylmethoxyimino) - 2 - (2 - formamidothiazol - 4 - yl)acetic acid (syn isomer, 7.9 g) under ice-cooling with stirring, and the stirring was continued at the same temperature for 30 minutes to prepare the activated acid solution. On the other hand, N-(Trimethylsilyl)acetamide (14.9 g) was added to a stirred suspension of 4-nitrobenzyl 7 - amino - 3 - chloro - 3 - cephem - 4 - carboxylate hydrochloride (6.6 g) in tetrahydrofuran (70 ml), and the stirring was continued at 38 to 43°C for 30 minutes. To the resultant solution was added the activated acid solution obtained above at −10.°C, followed by stirring at the same temperature for 30 minutes. To the reaction mixture were added water and ethyl acetate, and the separated organic layer was washed with a saturated aqueous sodium bicarbonate and water, and then dried over magnesium sulfate. Removal of the solvent gave 4-nitrobenzyl 7 - [2 - (benzhydryloxycarbonylmethoxyimino) - 2 - (2 - formamidothiazol - 4 - yl)acetamido] - 3 - chloro - 3 - cephem - 4 - carboxylate (syn isomer, 11.5 g).

IR (Nujol): 1780, 1725, 1680 $cm^{-1}$

NMR (DMSO-$d_6$, δ): 3.87 (2H, q, J=18.0Hz), 4.92 (2H, s), 5.33 (1H, d, J=4.0Hz), 5.46 (2H, s), 5.97 (1H, dd, J=4.0Hz, 8.0Hz), 6.88 (1H, s), 7.16—7.57 (11H, m), 7.67 (2H, d, J=8.0Hz), 8.20 (2H, d, J=8.0Hz), 8.50 (1H, s), 9.80 (1H, d, J=8.0Hz), 12.52 (1H, broad s)

The following compounds were obtained by reacting 7-aminocephem compounds with the corresponding acids according to a similar manner to that of Example 9.

Example 10

7 - [2 - (tert-Butoxycarbonylmethoxyimino) - 2 - (2 - formamidothiazol - 4 - yl)acetamido] - 3 - chloro - 3 - cephem - 4 - carboxylic acid (syn isomer).

IR (Nujol): 3160, 1780, 1720, 1670 $cm^{-1}$

Example 11

7 - [2 - (tert - Butoxycarbonylmethoxyimino) - 2 - (2 - formamidothiazol - 4 - yl)acetamido] - 3 - methoxy - 3 - cephem - 4 - carboxylic acid (syn isomer).

IR (Nujol): 3250, 1770, 1680, 1590, 1540 $cm^{-1}$

NMR (DMSO-$d_6$, δ): 1.47 (9H, z), 3.63 (2H, s), 3.78 (3H, s), 4.65 (2H, s), 5.18 (1H, d, J=5Hz), 5.62 (1H, d, d, J=5.8Hz), 7.53 (1H, s), 8.53 (1H, s), 9.56 (1H, d, J=8Hz)

Example 12

7 - [2 - (tert-Butoxycarbonylmethoxyimino) - 2 - (2 - aminothiazol - 4 - yl)acetamido] - 3 - chloro - 3 - cephem - 4 - carboxylic acid (syn isomer).

IR (Nujol): 1770, 1720, 1660, 1610 $cm^{-1}$

NMR (DMSO-$d_6$, δ): 1.44 (9H, s), 3,84 (2H, q, J=18.0Hz), 4.58 (2H, s), 5.29 (1H, d, J=5.0Hz), 5.85 (1H, dd, J=5.0Hz, 8.0Hz), 6.80 (1H, s), 7.27 (2H, broad-s), 9.57 (1H, d, J=8.0Hz)

Example 13

7 - [2 - (tert-Butoxycarbonylmethoxyimino) - 2 - (2 - aminothiazol - 4 - yl)acetamido] - 3 - methoxy - 3 - cephem - 4 - carboxylic acid (syn isomer).

IR (Nujol): 3350—3150, 1760, 1720 (shoulder), 1670, 1620, 1530 $cm^{-1}$

NMR (DMSO-$d_6$, δ): 1.45 (9H, s), 3.6 (2H, s), 3.78 (3H, s), 4.58 (2H, s), 5.15 (1H, d, J=5Hz), 5.57 (1H, d,d J=5.8Hz), 6.9 (1H,s) 9.43 (1H, d, J=8Hz)

Example 14

7 - [2 - (Benzylhydryloxycarbonylmethoxyimino) - 2 - (2 - formamidothiazol - 4 - yl)acetamido] - 3 - chloro - 3 - cephem - 4 - carboxylic acid (syn isomer).

IR (Nujol): 3170, 1770, 1730, 1670 $cm^{-1}$

NMR (DMSO-$d_6$, δ): 3.67 (2H, q, J=18.0Hz), 4.91 (2H, s), 5.27 (1H, d, J=5.0Hz), 5.88 (1H, dd, J=5.0Hz, 8.0Hz), 6.87 (1H, s), 7.14—7.60 (11H, m), 8.48 (1H, s), 9.74 (1H, d, J=8.0Hz), 12.59 (1H, broad s)

Example 15

Pivaloyloxymethyl 7 - [2 - (benzhydryloxycarbonylmethoxyimino) - 2 - (2 - aminothiazol - 4 - yl)acetamido] - 3 - chloro - 3 - cephem - 4 - carboxylate (syn isomer).

IR (Nujol): 3280, 3290, 1740 (broad), 1680, 1610 $cm^{-1}$

NMR (DMSO-$d_6$, δ): 1.14 (9H, s), 3.83 (2H, q, J=18.0Hz), 4.83 (2H, s), 5.28 (1H, d, J=4.0Hz), 5.73—6.06 (3H, m), 6.73 (1H, s), 6.84 (1H, s), 7.14—7.48 (10H, m), 9.63 (1H, d, J=8.0Hz)

Example 16

4-Nitrobenzyl 7 - [2 - benzhydryloxycarbonylmethoxyimino) - 2 - (2 - formamidothiazol - 4 - yl)acetamido] - 3 - chloro - 3 - cephem - 4 - carboxylate (syn isomer, 11.4 g) was dissolved in a mixed solution of methanol (40 ml), tetrahydrofuran (40 ml) and acetic acid (0.4 ml). After addition of 10% palladium carbon (5.7 g) thereto, the mixture was subjected to catalytic reduction in hydrogen stream at ambient temperature under atmospheric pressure. After removal of the catalyst by filtration, the filtrate was concentrated under reduced pressure. To the residue were added water and ethyl acetate, followed by adjusting to pH 7.5 with a saturated aqueous potassium carbonate. The separated aqueous layer was adjusted to pH 2.0 with 10% hydrochloric acid and extracted with ethyl acetate. The extract was washed with a saturated aqueous sodium chloride and then dried over magnesium sulfate. Removal of the solvent gave 7 - [2 - (benzhydryloxycarbonylmethoxyimino) - 2 - (2 - formamidothiazol - 4 - yl)ametamido] - 3 - chloro - 3 - cephem - 4 - carboxylic acid (syn isomer, 7.76 g).

IR (Nujol): 3170, 1770, 1730, 1670 $cm^{-1}$

NMR (DMSO-$d_6$, δ): 3.67 (2H, q, J=18.0Hz), 4.91 (2H, s), 5.27 (1H, d, J=5.0Hz), 5.88 (1H, dd, J=5.0Hz, 8.0Hz), 6.87 (1H, s), 7.14—7.60 (11H, m), 8.48 (1H, s), 9.74 (1H, d, J=8.0Hz), 12.59 (1H, broad s)

The following compounds were obtained by reducing the corresponding 4-nitrobenzyl ester of the cephem compounds with 10% palladium according to a similar manner to that of Example 16.

Example 17

7 - [2 - (tert - Butoxycarbonylmethoxyimino) - 2 - (2 - aminothiazol - 4 - yl)acetamido] - 3 - chloro - 3 - cephem - 4 - carboxylic acid (syn isomer).

IR (Nujol): 1770, 1720, 1660, 1610 $cm^{-1}$

NMR (DMSO-$d_6$, δ): 1.44 (9H, s), 3.84 (2H, q, J=18.0Hz), 4.58 (2H, s), 5.29 (1H, d, J=5.0Hz), 5.85 (1H, dd, J=5.0Hz, 8.0Hz), 6.80 (1H, s), 7.27 (2H, broad-s), 9.57 (1H, d, J=8.0Hz)

Example 18

7 - [2 - (tert-Butoxycarbonylmethoxyimino) - 2 - (2 - aminothiazol - 4 - yl)acetamido] - 3 - methoxy - 3 - cephem - 4 - carboxylic acid (syn isomer).

IR (Nujol): 3350—3150, 1760, 1720 (shoulder), 1670, 1620, 1530 $cm^{-1}$

NMR (DMSO-$d_6$, δ): 1.45 (9H, s), 3.6 (2H, s), 3.78 (3H, s), 4.58 (2H, s), 5.15 (1H, d, J=5Hz), 5.57 (1H, d,d, J=5.8Hz), 6.9 (1H, s), 9.43 (1H, d, J=8Hz)

Example 19

7 - [2 - Carboxymethoxyimino - 2 - (2 - aminothiazol - 4 - yl) - acetamido] - 3 - chloro - 3 - cephem - 4 - carboxylic acid (syn isomer).

IR (Nujol): 3250, 1770, 1660 $cm^{-1}$

NMR (DMSO-$d_6$, δ): 3.86 (2H, q, J=18.0Hz), 4.63 (2H, s), 5.29 (1H, d, J=4.5Hz), 5.86 (1H, dd, J=4.5Hz, 8.0Hz), 6.83 (1H, s), 9.59 (1H, d, J=8.0Hz)

Example 20

7 - [2 - Carboxymethoxyimino - 2 - (2 - aminothiazol - 4 - yl) - acetamido] - 3 - methoxy - 3 - cephem - 4 - carboxylic acid (syn isomer).

IR (Nujol): 1760, 1660 $cm^{-1}$

NMR (DMSO-$d_6$, δ): 3.67 (2H, s), 3.83 (3H, s), 4.73 (2H, s), 5.20 (1H, d, J=5Hz), 5.62 (1H, d,d, J=5, 8Hz), 7.05 (1H, s), 9.60 (1H, d, J=8Hz)

Example 21

Sodium 7 - [2 - benzhydryloxycarbonylmethoxyimino) - 2 - (2 - aminothiazol - 4 - yl)acetamido] - 3 - chloro - 3 - cephem - 4 - carboxylate (syn isomer).

IR (Nujol): 1750, 1660, 1600 (broad) $cm^{-1}$

NMR (DMSO-$d_6$, δ): 3.57 (2H, q, J=18.0Hz), 4.86 (2H, s), 5.13 (1H, d, J=5.0Hz), 5.66 (1H, dd, J=5.0Hz, 8.0Hz), 6.77 (1H, s), 6.88 (1H, s), 7.13—7.58 (10H, m), 9.55 (1H, d, J=8.0Hz)

Example 22

To a solution of 7 - [2 - (benzhydryloxycarbonylmethoxyimino) - 2 - (2 - formamidothiazol - 4 - yl)acetamido] - 3 - chloro - 3 - cephem - 4 - carboxylic acid (syn isomer, 7.6 g) in methanol (50 ml) and tetrahydrofuran (25 ml) was added conc. hydrochloric acid (2.4 g), and the mixture was stirred at ambient temperature for 3 hours. To the reduction mixture was added water (30 ml), and the resultant mixture was adjusted to pH 7.0 with a saturated aqueous sodium carbonate. After the organic solvent was removed, the resultant aqueous solution was stirred under ice-cooling. The precipitating solid was collected by filtration and washed with cold water and ethyl acetate, followed by drying over phosphorus pentoxide in vacuo to give sodium 7 - [2 - (benzhydryloxycarbonylmethoxyimino) - 2 - (2 - aminothiazol - 4 - yl) - acetamido] - 3 - chloro - 3 - cephem - 4 - carboxylate (syn isomer, 6.11 g).

IR (Nujol): 1750, 1660, 1600 (broad) $cm^{-1}$

NMR (DMSO-$d_6$, δ): 3.57 (2H, q, J=18.0Hz), 4.86 (2H, s), 5.13 (1H, d, J=5.0Hz), 5.66 (1H, dd, J=5.0Hz, 8.0Hz), 6.77 (1H, s), 6.88 (1H, s), 7.13—7.58 (10H, m), 9.55 (1H, d, J=8.0Hz)

The following compounds were obtained by reacting the cephem compounds having a formamido group with conc. hydrochloric acid according to a similar manner to that of Example 22.

Example 23

7 - [2 - Carboxymethoxyimino - 2 - (2 - aminothiazol - 4 - yl) - acetamido] - 3 - chloro - 3 - cephem - 4 - carboxylic acid (syn isomer).

IR (Nujol): 3250, 1770, 1660 $cm^{-1}$

NMR (DMSO-$d_6$, δ): 3.86 (2H, q, J=18.0Hz), 4.63 (2H, s), 5.29 (1H, d, J=4.5Hz), 5.86 (1H, dd, J=4.5Hz, 8.0Hz), 6.83 (1H, s), 9.59 (1H, d, J=8.0Hz)

Example 24

7 - [2 - Carboxymethoxyimino - 2 - (2 - aminothiazol - 4 - yl) - acetamido] - 3 - methoxy - 3 - cephem - 4 - carboxylic acid (syn isomer).

IR (Nujol): 1760, 1660 $cm^{-1}$

NMR (DMSO-$d_6$, δ): 3.67 (2H, s), 3.83 (3H, s), 4.73 (2H, s), 5.20 (1H, d, J=5Hz), 5.62 (1H, d,d J=5, 8Hz), 7.05 (1H, s), 9.60 (1H, d, J=8Hz)

Example 25

Pivaloyloxymethyl 7 - [2 - (benzhydryloxycarbonylmethoxyimino) - 2 - (2 - aminothiazol - 4 - yl)acetamido] - 3 - chloro - 3 - cephem - 4 - carboxylate (syn isomer).

IR (Nujol): 3280, 3190, 1740 (broad), 1680, 1610 $cm^{-1}$

NMR (DMSO-$d_6$, δ): 1.14 (9H, s), 3,83 (2H, q, J=18.0Hz), 4.83 (2H, s), 5.28 (1H, d, J=4.0Hz, 5.73—6.06 (3H, m), 6.73 (1H, s), 6.84 (1H, s), 7.14—7.48 (10H, m), 9.63 (1H, d, J=8.0Hz)

Example 26

Pivaloylmethyl 7 - [2 - carboxymethoxyimino - 2 - (2 - aminothiazol -4 - yl)acetamido] - 3 - chloro - 3 - cephem - 4 - carboxylate (syn isomer).

IR (Nujol): 3300, 1780, 1755, 1670 $cm^{-1}$

NMR (DMSO-$d_6$, δ): 1.16 (9H, s), 3.89 (2H, m), 4.58 (2H, s), 5.28 (1H, d, J=5.0Hz), 5.73—6.00 (3H, m), 6.75 (1H, s), 9.53 (1H, d, J=8.0Hz)

Example 27

To a solution of sodium 7 - [2 - (benzhydryloxycarbonylmethoxyimino) - 2 - (2 - aminothiazol - 4 - yl)acetamido] - 3 - chloro - 3 - cephem - 4 - carboxylate (syn isomer, 4.0 g) in N,N-dimethylformamide (40 ml) was added a solution of iodomethyl pivalate (1.5 g) in N,N-dimethylformamide (4.5 ml) at 0°C, and the mixture was stirred at the same temperature for 15 minutes. To the reaction were added ethyl acetate (150 ml) and water (100 ml), and the separated organic layer was washed with a saturated aqueous sodium bicarbonate and water, followed by drying over magnesium sulfate. Removal of the solvent gave

pivaloyloxymethyl 7 - [2 - (benzhydryloxycrbonylmethoxyimino) - 2 - (2 - aminothiazol - 4 - yl)acetamido] - 3 - chloro - - cephem - 4 - carboxylate (syn isomer, 3.91 g).

IR (Nujol): 3280, 3190, 1740 (broad), 1680, 1610 $cm^{-1}$

NMR ($DMSO-d_6$, δ): 1.14 (9H, s), 3.83 (2H, q, J=18.0Hz), 4.83 (2H, s), 5.28 (1H, d, J=4.0Hz), 5.73—6.06 (3H, m), 6.73 (1H, s), 6.84 (1H, s), 7.14—7.48 (10H, m), 9.63 (1H, d, J=8.0Hz)

Example 28

Trifluoracetic acid (5.8 g was added to a solution of pivaloylmethyl 7 - [2 - (benzhydryloxycarbonylmethoxyimino - 2 - (2 - aminothiazol - 4 - yl)acetamido] - 3 - chloro - 3 - cephem - 4 - carboxylate (syn isomer, 3.8 g) in methylene chloride (15.2 ml) and anisole (1.1 g) under ice-cooling, and the mixture was stirred at ambient temperature for an hour. To the reaction mixture was added diisopropyl ether with stirring, and the precipitating solid was collected by filtration, followed by washing with diisopropyl ether. This solid was added to a mixture of ethyl acetate and water, followed by adjusting to pH 7.5 with a saturated aqueous potassium carbonate.

The separated aqueous solution was further adjusted to pH 2.5 with 10% hydrochloric acid, and then the precipitating substance was collected by filtration and washed with water, followed by drying over phosphorus pentoxide in vacuo to give pivaloyloxymethyl 7 - [2 - carboxymethoxyimino - 2 - (2 - aminothiazol - 4 - yl)acetamido] - 3 - chloro - 3 - cephem - 4 - carboxylate (syn isomer, 2.36 g).

IR (Nujol): 3300, 1780, 1755, 1670 $cm^{-1}$

NMR ($DMSO-d_6$, δ): 1.16, (9H, s), 3.89 (2H, m), 4.58 (2H, s), 5.28 (1H, d, J=5.0Hz), 5.73—6.00 (3H, m), 6.75 (1H, s), 9.53 (1H, d, J=8.0Hz)

**Claims for the Contracting States: BE CH DE FR GB IT LI LU NL SE**

1. A compound of the formula:

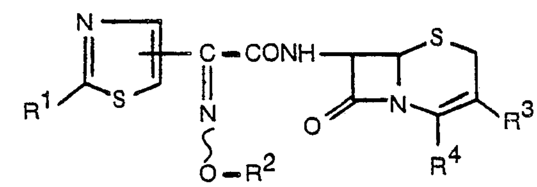

(I)

wherein

$R^1$ is amino or protected amino

$R^2$ is carboxy($C_{1-6}$)alkylene or protected carboxy($C_{1-6}$)alkylene,

$R^3$ is halogen or $C_{1-6}$ alkoxy, and

$R^4$ is carboxy or protected carboxy, and a pharmaceutically acceptable salt thereof.

2. A syn isomer of the compound of claim 1.

3. A compound of claim 2, wherein $R^1$ is amino optionally protected by acyl, $R^2$ is carboxy($C_{1-6}$)alkylene optionally protected by an ester group, $R^3$ is halogen or $C_{1-6}$ alkoxy, and $R^5$ is carboxy optionally protected by an ester group.

4. A compound of claim 3, wherein $R^1$ is amino or $C_{1-6}$ alkanoylamino, $R^2$ is carboxy($C_{1-6}$)alkylene, $C_{1-6}$ alkoxycarbonyl($C_{1-6}$)alkylene, or mono- or di- or triphenyl($C_{1-6}$alkoxycarbonyl($C_{1-6}$)alklene, $R^3$ is chloro or methoxy, and $R^4$ is carboxy, nitrophenyl($C_{1-6}$)alkoxycarbonyl or $C_{1-6}$ alkanoyloxy($C_{1-6}$)alkoxycarbonyl.

5. A compound of claim 4, which is 7 - [2 - carboxymethoxyimino - 2 - (2 - aminothiazol - 4 - yl) - acetamido] - 3 - chloro - 3 - cephem - 4 - carboxylic acid (syn isomer).

6. A compound of claim 4, which is 7 - [2 - carboxymethoxyimino - 2 - (2 - aminothiazol - 4 - yl)acetamido] - 3 - methoxy - 3 - cephem - 4 - carboxylic acid (sun isomer).

7. A compound of claim 4, which is pivaloyloxymethyl 7 - [2 - carboxymethoxyimino - 2 - (2 - aminothiazol - 4 - yl) - acetamido] - 3 - chloro - 3 - cephem - 4 - carboxylate (syn isomer).

8. A process for preparing a compound of claim 1, which comprises

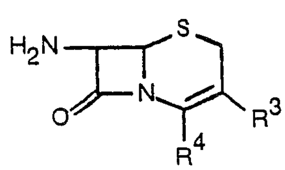

(II)

wherein $R^3$ and $R^4$ are each as defined in claim 1, or its reactive derivative at the amino group or a salt thereof, with a carboxylic acid of the formula:

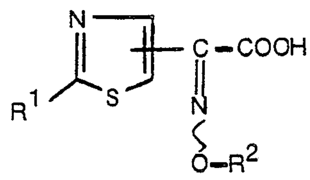

(III)

wherein $R^1$ and $R^2$ are each as defined in claim 1, or its reactive derivative at the carboxy group or a salt thereof, or

(2) subjecting a compound of the formula:

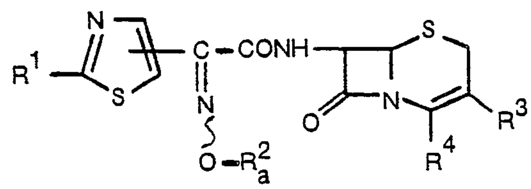

(Ia)

wherein $R_a^2$ is protected carboxy($C_{1-6}$)alkylene, and

$R^1$, $R^3$ and $R^4$ are each as defined in claim 1, or a salt thereof, to elimination reaction of the carboxy-protective group on the $R_a^2$, to provide a compound of the formula:

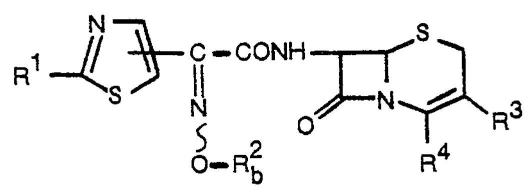

(Ib)

wherein $R_b^2$ is carboxy($C_{1-6}$)alkylene, and

$R^1$, $R^3$ and $R^4$ are each as defined in claim 1, or a salt thereof, or

(3) subjecting a compound of the formula:

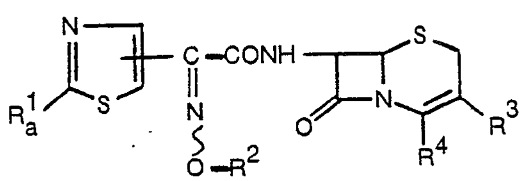

(Ic)

wherein $R_a^1$ is protected amino, and

$R^2$, $R^3$ and $R^4$ are each as defined in claim 1, or a salt thereof, to elimination reaction of the amino-protective group on the $R_a^1$, to provide a compound of the formula:

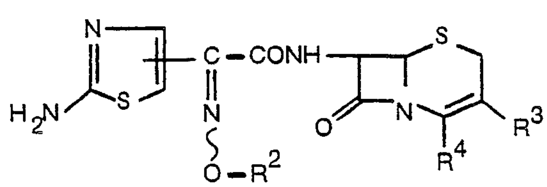

(Id)

wherein $R^2$, $R^3$ and $R^4$ are each as defined in claim 1, or a salt thereof, or

(4) subjecting a compound of the formula:

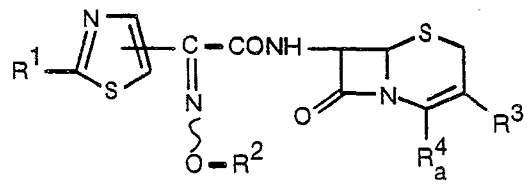

(Ie)

wherein $R^4_a$ is protected carboxy, and

$R^1$, $R^2$ and $R^3$ are each as defined in claim 1, or a salt thereof, to elimination reaction of the carboxy-protective group on the $R^4_a$, to provide a compound of the formula:

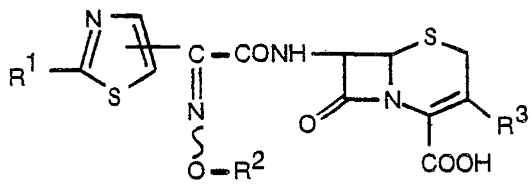

(If)

wherein $R^1$, $R^2$ and $R^3$ are each as defined in claim 1, or a salt thereof, or

(5) subjecting a compound of the formula:

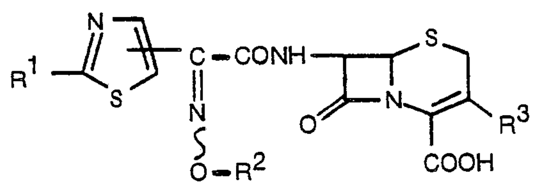

(If)

wherein $R^1$, $R^2$ and $R^3$ are each as defined in claim 1, or a salt thereof to introduction reaction of the carboxy-protective group, to provide a compound of the formula:

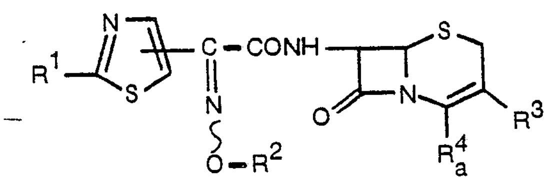

(Ie)

wherein R 04, $R^2$ and $R^3$ are each as defined in claim 1 and $R^4_a$ is as defined above, or a salt thereof.

9. A pharmaceutical composition comprising a compound of the formula:

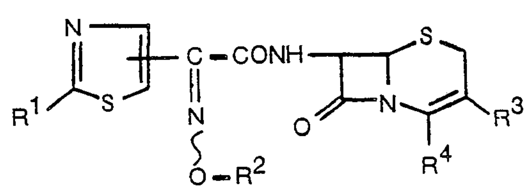

(I)

wherein,

$R^1$ is amino,

$R^2$ is carboxy $(C_{1-6})$alkylene,

$R^3$ is halogen or $C_{1-6}$ alkoxy, and

$R^4$ is carboxy

or pharmaceutically acceptable salt thereof in association with a pharmaceutically acceptable, substantially non-toxic carrier or excipient.

10. A compound of formula (I) as defined in claim 9 for use as an active therapeutic substance.

11. A compound of formula (I) as defined in claim 9 for use as an active substance for treating infectious diseases caused by pathogenic microorganisms.

**Claim for the Contracting State: AT**

A process for preparing a compound of the formula:

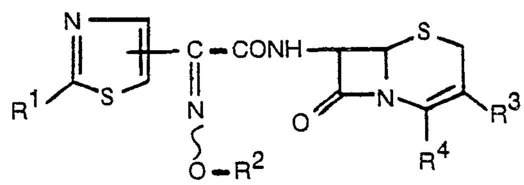

(I)

wherein
$R^1$ is amino or protected amino,
$R^2$ is carboxy($C_{1-6}$)alkylene or protected carboxy($C_{1-6}$)alkylene,
$R^3$ is halogen or $C_{1-6}$ alkoxy, and
$R^4$ is carboxy or protected carboxy, and a pharmaceutically acceptable salt thereof, which comprises
(1) reacting a 7-amino-3-cephem compound of the formula:

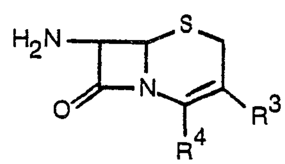

(II)

wherein $R^3$ and $R^4$ are each as defined above, or its reactive derivative at the amino group or a salt thereof, with a carboxylic acid of the formula:

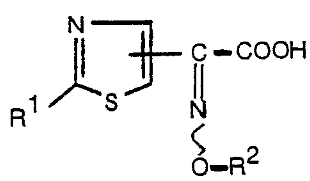

(III)

wherein $R^1$ and $R^2$ are each as defined above, or its reactive derivative at the carboxy group or a salt thereof, or
(2) subjecting a compound of the formula:

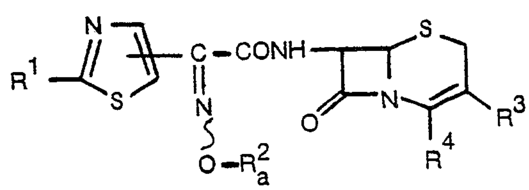

(Ia)

wherein $R^2_a$ is protected carboxy($C_{1-6}$)alkylene, and
$R^1$, $R^3$ and $R^4$ are each as defined above, or a salt thereof, to elimination reaction of the carboxy-protective group on the $R^2_a$, to provide a compound of the formula:

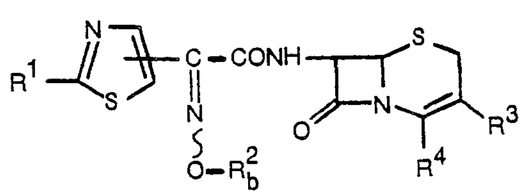

(Ib)

wherein $R_b^2$ is carboxy($C_{1-6}$)alkylene, and $R^1$, $R^3$ and $R^4$ are each as defined above, or a salt thereof, or

(3) subjecting a compound of the formula:

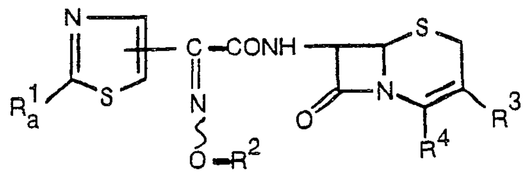

(Ic)

wherein $R_a^1$ is protected amino, and $R^2$, $R^3$ and $R^4$ are each as defined above, or a salt thereof, to elimination reaction of the amino-protective group on the $R_a^1$, to provide a compound of the formula:

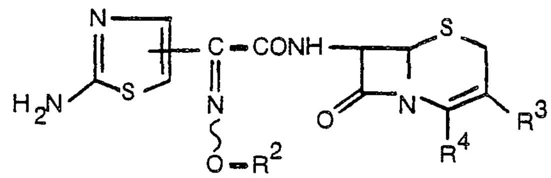

(Id)

wherein $R^2$, $R^3$ and $R^4$ are each as defined above, or a salt thereof, or

(4) subjecting a compound of the formula:

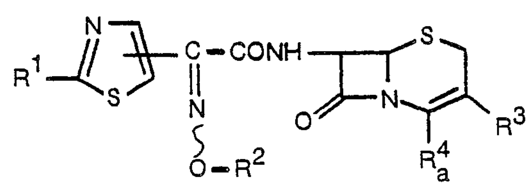

(Ie)

wherein $R_a^4$ is protected carboxy, and $R^1$, $R^2$ and $R^3$ are each as defined in above, or a salt thereof, to elimination reaction of the carboxy-protective group on the $R_a^4$, to provide a compound of the formula:

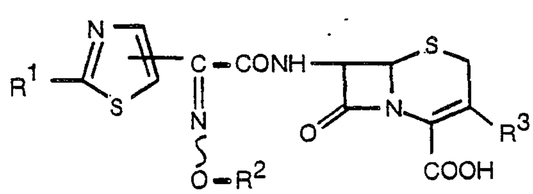

(If)

wherein $R^1$, $R^2$ and $R^3$ are each as defined above, or a salt thereof, or

(5) subjecting a compound of the formula:

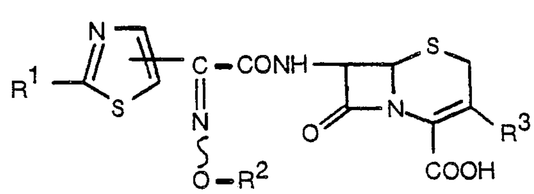

(If)

wherein $R^1$, $R^2$ and $R^3$ are each as defined above or a salt thereof to introduction reaction of the carboxy-protective group, to provide a compound of the formula:

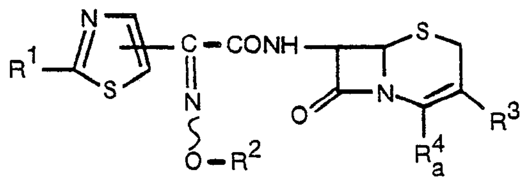

(Ie)

wherein $R^1$, $R^2$, $R^3$ and $R^4_a$ are as defined above, or a salt thereof.

**Patentansprüche für die Vertragsstaaten: LI BE CH DE FR GB IT LU NL SE**

1. Eine Verbindung mit der Formel

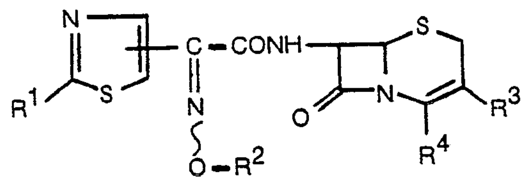

worin
$R^1$ Amino oder geschütztes Amimo ist,
$R^2$ Carboxy-($C_{1-6}$)-alkylen oder geschütztes Carboxy-($C_{1-6}$)alkylen ist,
$R^3$ Halogen oder $C_{1-6}$-Alkoxy ist, und
$R^4$ Carboxy oder geschütztes Carboxy ist, und ein pharmazeutisch brauchbares Salz davon.

2. Ein syn-Isomeres der Verbindung nach Anspruch 1.

3. Eine Verbindung nach Anspruch 2, worin $R^1$ Amino, gegebenenfalls geschützt durch Acyl ist, $R^2$ Carboxy-($C_{1-6}$)-alkylen, gegebenenfalls geschützt durch eine Estergruppe, ist, $R^3$ Halogen oder $C_{1-6}$-Alkoxy ist, und $R_1$ Carboxy, gegebenenfalls geschützt durch eine Estergruppe, ist.

4. Eine Verbindung nach Anspruch 3, worin $R^1$ Amino oder $C_{1-6}$-Alkanoylamino ist, $R^2$ Carboxy-($C_{1-6}$)alkylen, $C_{1-6}$-Alkoxycarbonyl-($C_{1-6}$)-alkylen oder Mono- oder Di- oder Triphenyl-($C_{1-6}$)-alkoxycarbonyl-($C_{1-6}$)alkylen ist, $R^3$ Chlor oder Methoxy ist und $R^4$ Carboxy, Nitrophenyl-($C_{1-6}$)-alkoxycarbonyl oder $C_{1-6}$-Alkanoyloxy-($C_{1-6}$)alkoxycarbonyl ist.

5. Eine Verbindung nach Anspruch 4, nämlich 7 - [2 - Carboxymethoxyimino - 2 - (2 - aminothiazol - 4 - yl) - acetamido] - 3 - chlor - 3 - cephem - 4 - carbonsäre (syn-Isomeres).

6. Eine Verbindung nach Anspruch 4, nämlich 7 - [2 - Carboxymethoxyimino - 2 - (2 - aminothiazol - 4 - yl) - acetamido] - 3 - methoxy - 3 - cephem - 4 - carbonsäure (syn-Isomeres).

7. Eine Verbindung nach Anspruch 4, nämlich Pivaloyloxymethyl - 7 - [2 - carboxymethoxyimino - 2 - (2 - aminothiazol - 4 - yl) - acetamido] - 3 - chlor - 3 - carboxylat (syn-Isomeres).

8. Ein Verfahren zur Herstellung einer Verbindung nach Anspruch 1, welches umfaßt:
(1) die Reaktion einer 7 - Amino - 3 - cephem - verbindung der Formel

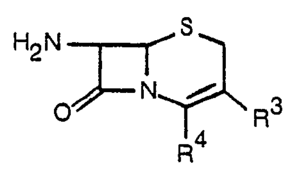

(II)

worin $R^3$ und $R^4$ jeweils wie in Anspruch 1 definiert sind, oder ihres reaktiven Derivats an der Aminogruppe, oder eines Salzez davon, mit einer Carbonsäre der Formel

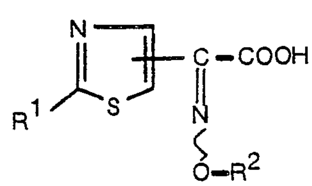

(III)

worin $R^1$ und $R^2$ jeweils wie in Anspruch 1 definiert sind, oder ihrem reaktivem Derivat an der Carboxygruppe, oder einem Salz davon, oder

(2) Unterwerfen einer Verbindung der Formel

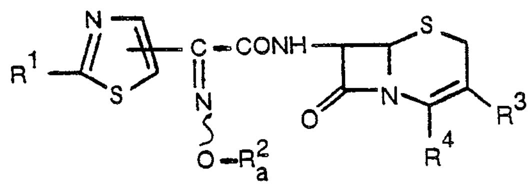

(Ia)

worin $R^2_a$ geschütztes Carboxy-($C_{1-6}$)-alkylen ist und $R^1$, $R^3$ und $R^4$ jeweils wie in Anspruch 1 definiert sind, oder eines Salzes davon, der Eliminierungsreaktion der Carboxyschutzgruppe am $R^2_a$, unter Bindung einer Verbindung der Formel

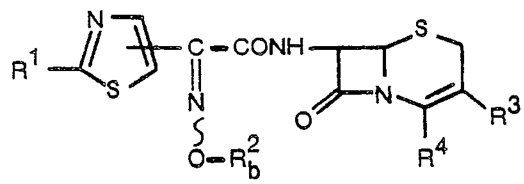

(Ib)

worin $R^2_b$ Carboxy-($C_{1-6}$)-Alkylen ist und $R^1$, $R^3$ und $R^4$ jeweils wie in Anspruch 1 definiert sind, oder eines Salzes davon, oder

(3) Unterwerfen einer Verbindung der Formel

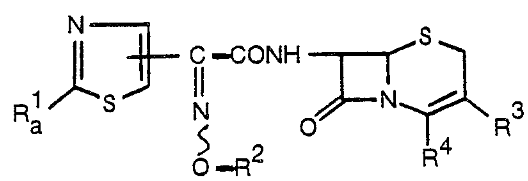

(Ic)

worin $R^1_a$ gechütztes Amino ist, und $R^2$, $R^3$ und $R^4$ jeweils wie in Anspruch 1 definiert sind, oder eines Salzes davon, der Eliminierungsreaktion der Aminoschutzgruppe an dem $R^1_a$, unter Bildung einer Verbindung der Formel

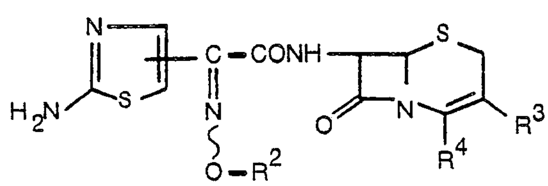

(Id)

worin $R^2$, $R^3$ und $R^4$ jeweils wie in Anspruch 1 definiert sind, oder eines Salzes davon, oder

(4) Unterwerfen einer Verbindung der Formel

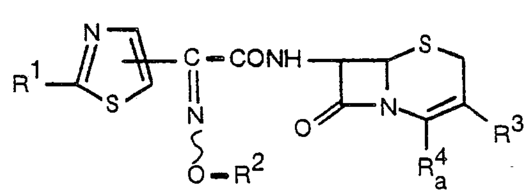

(Ie)

worin $R_a^4$ geschütztes Carboxy ist und $R^1$, $R^2$ und $R^3$ jeweils wie in Anspruch 1 definiert sind, oder eines Salzes davon, der Eliminierungsreaktion für die Carboxyschutzgruppe am $R_a^4$, unter Bildung einer Verbindung der Formel

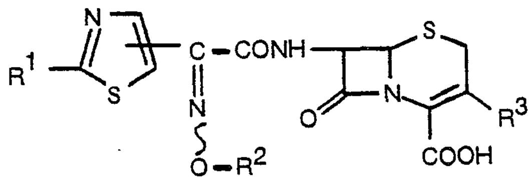

(If)

worin $R^1$, $R^2$ und $R^3$ jeweils wie in Anspruch 1 definiert sind, oder eines Salzes davon, oder

(5) Unterwerfen einer Verbindung der Formel

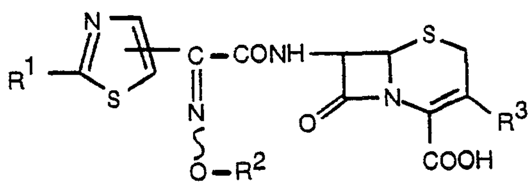

(If)

worin $R^1$, $R^2$ und $R^3$ jeweils wie in Anspruch 1 definiert sind, oder eines Salzes davon, der Einführungsreaktion für die Carboxyschutzgruppe unter Bildung einer Verbindung der Formel

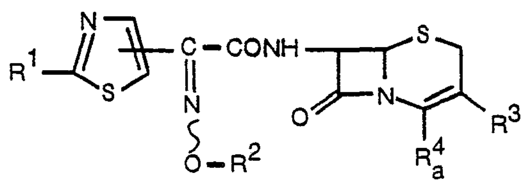

(Ie)

worin $R^1$, $R^2$ und $R^3$ jeweils wie in Anspruch 1 definiert sind und $R_a^4$ wie vorstehend definiert ist, oder eines Salzes davon.

9. Eine pharmazeutische Zusammensetzung, umfassend eine Verbindung der Formel

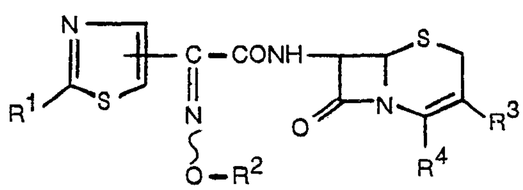

(I)

worin

$R^1$ Amino ist,

$R^2$ Carboxy-($C_{1-6}$)-alkylen ist,

$R^3$ Halogen oder $C_{1-6}$-Alkoxy ist und

$R^4$ Carboxy ist

oder ein pharmazeutisch brauchbares Salz davon, zusammen mit einem pharmazeutisch brauchbaren, im wesentlichen nichttoxischen Träger oder Exzipienten.

10. Eine Verbindung der Formel (I), wie in Anspruch 9 definiert, zur Verwendung als eine aktive therapeutische Substanz.

11. Eine Verbindung der Formel (I), wie in Anspruch 9 definiert, zur Verwendung als eine aktive Substanz zur Behandlung von Infektionserkrankungen, die durch pathogene Mikroorganismen bewirkt werden.

**Patentanspruch für den Vertragsstaat: AT**

Ein Verfahren zur Herstellung einer Verbindung der Formel

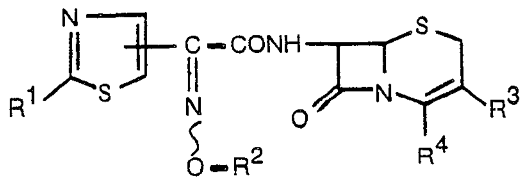

(I)

worin

$R^1$ Amino oder geschütztes Amino ist,

$R^2$ Carboxy-($C_{1-6}$)-alkylen oder geschütztes Carboxy-($C_{1-6}$)-alkylen ist,

$R^3$ Halogen oder $C_{1-6}$-Alkoxy ist, und

$R^4$ Carboxy oder geschütztes Carboxy ist,

und eines pharmazeutisch braunchbaren Salzes davon, welches unfaßt

(1) die Reaktion einer 7 - Amino - 3 - cephem - verbindung der Formel

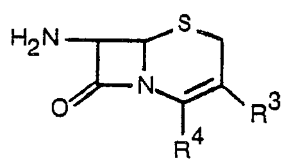

(II)

worin $R^3$ und $R^4$ jeweils wie vorstehend definiert sind, oder ihres reaktiven Derivats an der Aminogruppe, oder eines Salzes davon, mit einer Carbonsäure der Formel

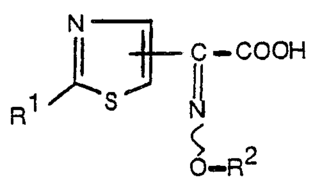

(III)

worin $R^1$ und $R^2$ jeweils wie vorstehend definiert sind, oder ihrem reaktivem Derivats an der Carboxygruppe, oder eines Salzes davon, oder

(2) Unterwerfen einer Verbindung der Formel

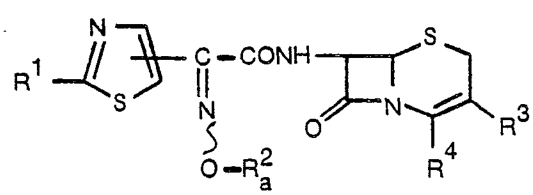

(Ia)

worin $R^2_a$ geschütztes Carboxy-($C_{1-6}$)-alkylen ist und $R^1$, $R^3$ und $R^4$ jeweils wie vorstehend definiert sind, oder eines Salzes davon, der Eliminierungsreaktion der Carboxyschutzgruppe am $R^2_a$, unter Bindung einer Verbindung der Formel

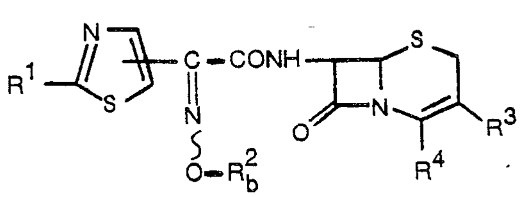

(Ib)

worin $R_b^2$ Carboxy-$(C_{1-6})$-Alkylen ist und $R^1$, $R^3$ und $R^4$ jeweils wie vorstehend definiert sind, oder eines Salzes davon, oder

(3) Unterwerfen einer Verbindung der Formel

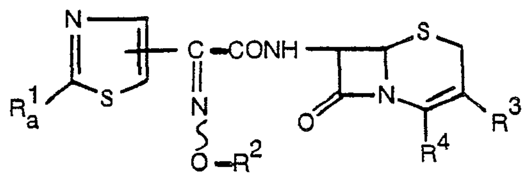

(Ic)

worin $R_a^1$ geschütztes Amino ist, und $R^2$, $R^3$ und $R^4$ jeweils wie vorstehend definiert sind, oder eines Salzes davon, der Eliminierungsreaktion der Aminoschutzgruppe an dem $R_a^1$, unter Bildung einer Verbindung der Formel

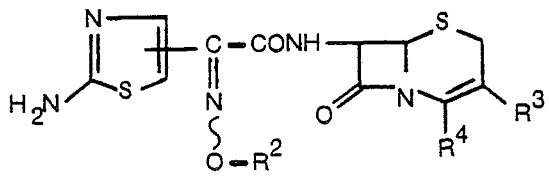

(Id)

worin $R^2$, $R^3$ und $R^4$ jeweils wie vorstehend definiert sind, oder eines Salzes davon, oder

(4) Unterwerfen einer Verbindung der Formel

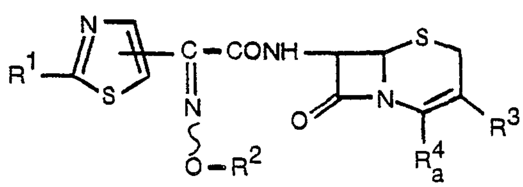

(Ie)

worin $R_a^4$ geschütztes Carboxy ist und $R^1$, $R^2$ und $R^3$ jeweils wie vorstehend definiert sind, oder eines Salzes davon, der Eliminierungsreaktion für die Carboxyschutzgruppe am $R_a^4$, unter Bildung einer Verbindung der Formel

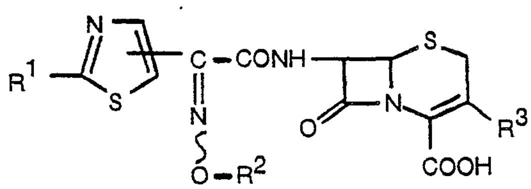

(If)

worin $R^1$, $R^2$ und $R^3$ jeweils wie vorstehend definiert sind, oder eines Salzes davon, oder

(5) Unterwerfen einer Verbindung der Formel

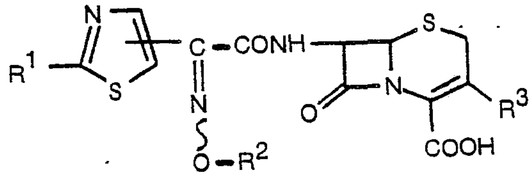

(If)

worin $R^1$, $R^2$ und $R^3$ jeweils wie vorstehend definiert sind, oder eines Salzes davon, der Einführungsreaktion für die Carboxyschutzgruppe unter Bildung einer Verbindung der Formel

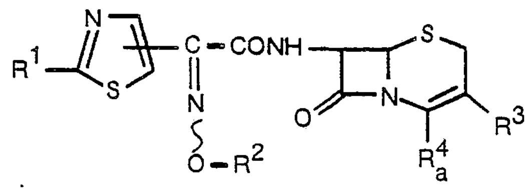

(Ie)

worin $R^1$, $R^2$, $R^3$ und $R^4_a$ jeweils wie vorstehend definiert sind, oder eines Salzes davon.

**Revendications pour les Etats contractants: BE CH DE FR GB IT LI LU NL SE**

1. Composé répondant à la formule:

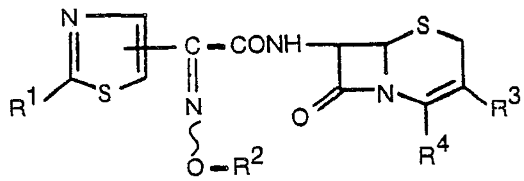

dans laquelle
$R^1$ est un groupe amino ou amino protégé,
$R^2$ est un groupe carboxy(alkylène en $C_1$ à $C_6$) ou carboxy(alkylène en $C_1$ à $C_6$) protégé,
$R^3$ est un halogène ou un groupe alcoxy en $C_1$ à $C_6$; et
$R^4$ est un groupe carboxy ou carboxy protégé,
et un de ses sels pharmaceutiquement acceptables.

2. Isomère syn du composé de la revendiction 1.

3. Composé selon la revendiction 2, dans lequel $R^1$ est un groupe amino, protégé si on le désire par groupe acyle, $R^2$ est un groupe carboxy(alkylène en $C_1$ à $C_6$) protégé si on le désire par un groupe ester; $R^3$ est un halogène ou un groupe alcoxy en $C_1$ à $C_6$, et $R^4$ est un groupe carboxy, protégé si on le désire par un groupe ester.

4. Composé selon la revendication 3, dans lequel $R^1$ est un groupe amino ou (alcanoyl en $C_1$ à $C_6$)amino, $R^2$ est un groupe carboxy(alkylène en $C_1$ à $C_6$), (alcoxy en $C_1$ à $C_6$) carbonyle-(alkylène en $C_1$ à $C_6$), ou mono- ou di- ou triphényl-(alcoxy en $C_1$ à $C_6$)carbonyle(alkylène en $C_1$ à $C_6$); $R^3$ est un chloro ou un groupe méthoxy, et $R^4$ est un groupe carboxy, nitrophényl(alcoxy en $C_1$ à $C_6$)carbonyle ou (alcanoyloxy en $C_1$ à $C_6$)(alcoxy en $C_1$ à $C_6$)carbonyle.

5. Composé selon la revendication 4, qui est l'acide 7 - [2 - carboxyméthoxyimino - 2 - (2 - aminothiazol 4 - yl) - acétamido] - 3 - chloro - 3 - céphème - 4 - carboxylique (isomère syn).

6. Composé selon la revensication 4, qui est l'acide 7 - [2 - carboxyméthoxyimino - 2 - (2 - aminothiazol - 4 - yl)acétamido] - 3 - méthoxy - 3 - céphème - 4 - carboxylique (isomère syn).

7. Composé selon la revendication 4, qui est le 7 - [2 - carboxyméthoxyimino - 2 - (2 - aminothiazol - 4 - yl) - acétamido] - 3 - chloro - 3 - céphème - 4 - carboxylate de pivaloyloxyméthyle (isomère syn).

8. Procédé de préparation d'un composé selon la revendication 1, qui consiste:
(1) à faire réagir un 7-amino-3-céphème répondant à la formule:

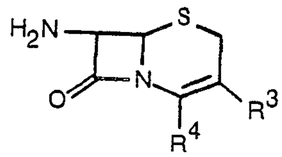

(II)

dans laquelle

$R^3$ et $R^4$ sont chacun tels que définis dans la revendication 1, ou son dérivé réactif sur le groupe amino ou un de ses sels avec un acide carboxylique répondant à la formule:

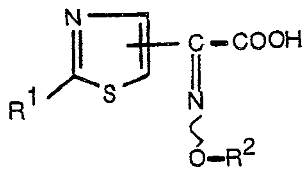

(III)

dans laquelle

$R^1$ et $R^2$ sont chacun tels que définis dans la revendication 1, ou son dérivé réactif sur le groupe carboxy ou un de ses sels, ou

(2) à soumettre un composé répondant à la formule:

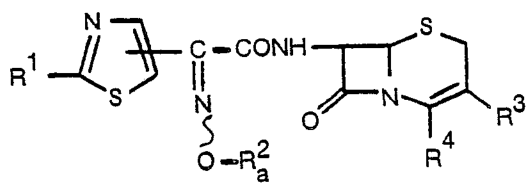

(Ia)

dans laquelle

$R^2_a$ est un groupe carboxy(alkylène en $C_1$ à $C_6$) protégé, et

$R^1$, $R^3$ et $R^4$ sont chacun tels que définis dans la revendication 1, ou un de ses sels, à une réaction d'élimination du groupe protecteur du carboxy sur le radical $R^2_a$, pour donner un composé répondant à la formule:

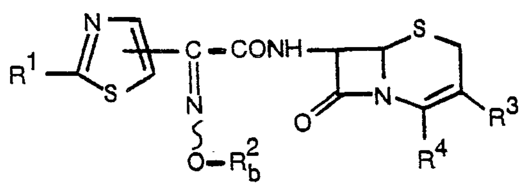

(Ib)

dans laquelle

$R^2_b$ est un groupe carboxy (alkylène en $C_1$ à $C_6$) et

$R^1$, $R^3$ et $R^4$ sont chacun tels que définis dans la revendication 1 ou un de ses sels, or

(3) à soumettre un composé répondant à la formule:

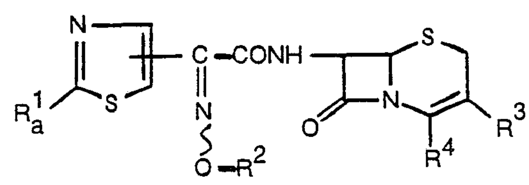

(Ic)

dans laquelle

$R^1_a$ est un groupe amino protégé, et

$R^2$, $R^3$ et $R^4$ sont chacun tels que définis dans la revendication 1 ou un de ses sels, à une réaction d'élimination du groupe protecteur de l'amino sur le groupe $R^1_a$, pour donner un composé répondant à la formule:

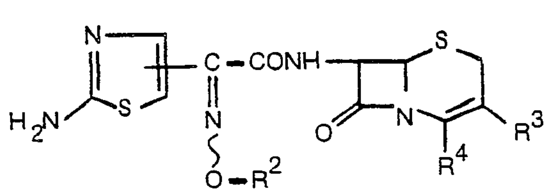

(Id)

dans laquelle

$R^2$, $R^3$ et $R^4$ sont chacun tels que définis dans la revendication 1, ou un de ses sels ou

(4) à soumettre un composé répondant à la formule:

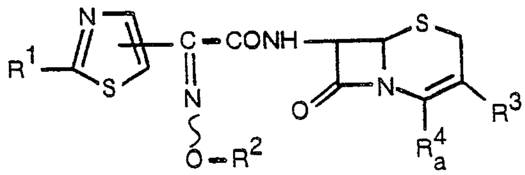

(Ie)

où $R_a^4$ est un groupe carboxy protégé, et

$R^1$, $R^2$ et $R^3$ sont chacun tels que définis dans la revendication 1 ou un de ses sels, à une réation d'élimination du groupe protecteur du carboxy sur le radical $R_a^4$, pour donner un composé répondant à la formule:

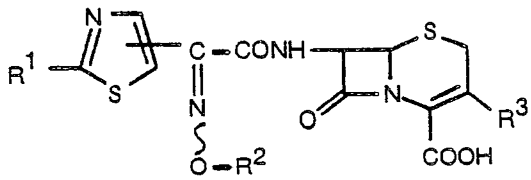

(If)

dans laquelle

$R^1$, $R^2$ et $R^3$ sont chacun tels que définis dans la revendication 1, ou un de ses sels, ou

(5) à soumettre un composé répondant à la formule:

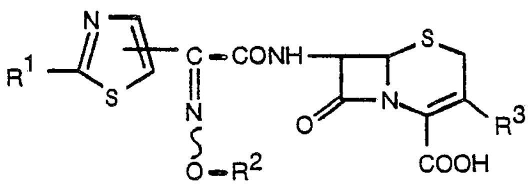

(If)

dans laquelle

$R^1$, $R^2$ et $R^3$ sont chacun tels que définis dans la revendication 1, ou un de ses sels, à une réction d'introduction du groupe protecteur du carboxy, pour donner un composé répondant à la formule:

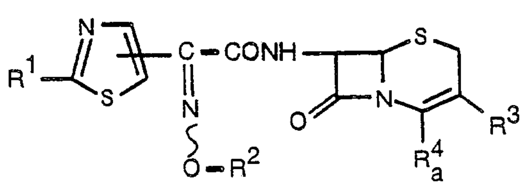

(Ie)

dans laquelle

$R^1$, $R^2$ et $R^3$ sont chacun tels que définis dans la revendication 1, et $R_a^4$ est tel que défini ci-dessus ou un de ses sels.

9. Composition pharmaceutique comprenant un composé répondant à la formule:

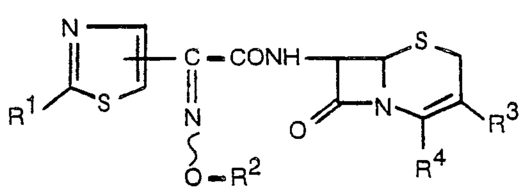

(I)

dans laquelle

$R^1$ est un groupe amino

$R^2$ est un groupe carboxy(alkylène en $C_1$ à $C_6$),

$R^3$ est un halogène ou un groupe(alcoxy en $C_1$ à $C_6$), et

$R^4$ est un groupe carboxy

ou un de ses sels pharmaceutiquement acceptables en association avec un support ou excipient pharmaceutiquement acceptable, pratiquement non toxique.

10. Composé répondant à la formule (I) telle que définie dans la revendication 9 pour l'utilisation comme substance thérapeutique active.

11. Composé répondant à la formule (I) telle que définie dans la revendication 9 pour l'utilisation comme substance active pour traiter des maladies infectieuses provoquées par des microoganisms pathogènes.

**Revendication pour l'Etat contractant: AT**

1. Procédé pour préparer un composé répondant à la formule:

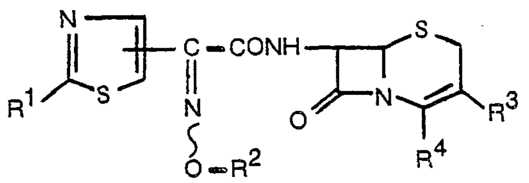

(I)

dans laquelle

$R^1$ est un groupe amino ou amino protégé,

$R^2$ est un groupe carboxy(alkylène en $C_1$ à $C_6$) ou un groupe carboxy(alkylène en $C_1$ à $C_6$)protégé,

$R^3$ est un halogène ou un groupe alcoxy en $C_1$ à $C_6$, et

$R^4$ est un groupe carboxy ou carboxy protégé,

et un de ses sels pharmaceutiquement acceptables, qui consiste:

(1) à faire réagir un 7-amino-3-céphème répondant à la formule:

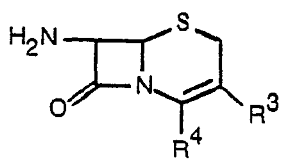

(II)

dans laquelle

$R^3$ et $R^4$ sont chacun tels que définis ci-dessus, ou son dérivé réactif sur le groupe amino ou un de ses sels, avec un acide carboxylique répondant à la formule:

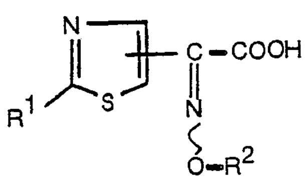

(III)

dans laquelle

$R^1$ et $R^2$ sont chacun tels que définis ci-dessus, ou son dérivé réactif sur le groupe carboxy ou un de ses sels, ou

(2) à soumettre un composé répondant à la formule:

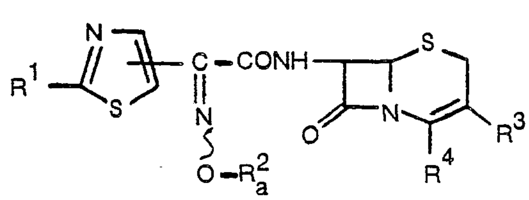

(Ia)

dans laquelle

$R^2_a$ est un groupe carboxy(alkylène en $C_1$ à $C_6$) protégé, et

$R^1$, $R^3$ et $R^4$ sont chacun tels que définis ci-dessus, ou un de ses sels, à une réaction d'élimination du groupe protecteur du carboxy sur le radical $R^2_a$, pour donner un composé répondant à la formule:

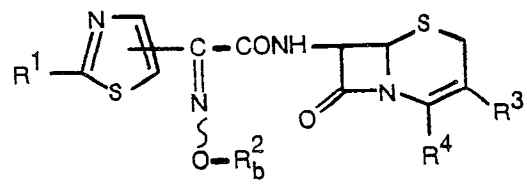

(Ib)

dans laquelle

$R^2_b$ est un groupe carboxy (alkylène en $C_1$ à $C_6$) et

$R^1$, $R^3$ et $R^4$ sont chacun tels que définis ci-dessus ou un de ses sels, ou

(3) à soumettre un composé répondant à la formule:

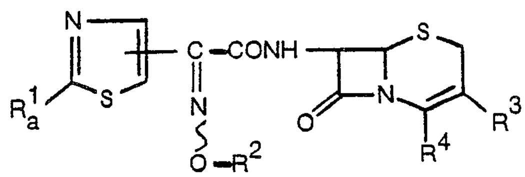

(Ic)

dans laquelle

$R^1_a$ est un groupe amino protégé, et

$R^2$, $R^3$ et $R^4$ sont chacun tels que définis ci-dessus, ou un de ses sels, à une réaction d'élimination du groupe protecteur de l'amino sur le groupe $R^1_a$, pour donner un composé répondant à la formule:

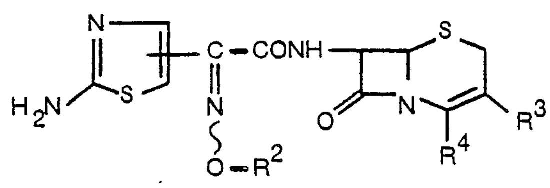

(Id)

dans laquelle

$R^2$, $R^3$ et $R^4$ sont chacun tels que définis ci-dessus, ou un de ses sels, ou

(4) à soumettre un composé répondant à la formule:

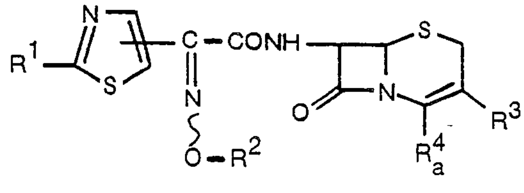

(Ie)

où $R^4_a$ est un groupe carboxy protégé, et

$R^1$, $R^2$ et $R^3$ sont chacun tels que définis ci-dessus, ou un de ses sels, à une réaction d'élimination du groupe protecteur du carboxy sur le radical $R^4_a$, pour donner un composé répondant à la formule:

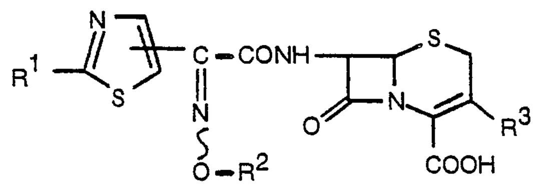

(If)

dans laquelle

$R^1$, $R^2$ et $R^3$ sont chacun tels que définis ci-dessus, ou un de ses sels, ou

(5) à soumettre un composé répondant à la formule:

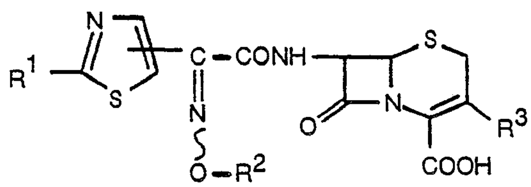

(If)

dans laquelle

$R^1$, $R^2$ et $R^3$ sont chacun tels que définis ci-dessus, ou un de ses sels, à une réaction d'introduction du groupe protecteur du carboxy, pour donner un composé répondant à la formule:

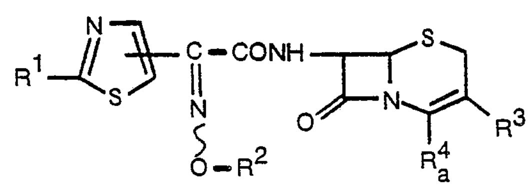

(Ie)

dans laquelle

$R^1$, $R^2$, $R^3$ et $R^4_a$ sont chacun tels que définis ci-dessus ou un de ses sels.